(19) Europäisches Patentamt
European Patent Office
Office européen des brevets

(11) Veröffentlichungsnummer: **0 315 008 B1**

(12) **EUROPÄISCHE PATENTSCHRIFT**

(45) Veröffentlichungstag der Patentschrift: **09.09.92**

(51) Int. Cl.[5]: **C07C 69/712**, C07C 67/31, C07C 43/295, C07C 41/16, A01N 39/02, C07D 213/79, C07D 213/84, C07D 213/30, C07C 255/16, C07C 59/135, C07C 235/32

(21) Anmeldenummer: **88117666.3**

(22) Anmeldetag: **24.10.88**

Die Akte enthält technische Angaben, die nach dem Eingang der Anmeldung eingereicht wurden und die nicht in dieser Patentschrift enthalten sind.

(54) **(6-(Hetero)Aryloxy-naphthalin-2-yl-oxy)-alkancarbonsäure-Derivate.**

(30) Priorität: **03.11.87 DE 3737179**

(43) Veröffentlichungstag der Anmeldung: **10.05.89 Patentblatt 89/19**

(45) Bekanntmachung des Hinweises auf die Patenterteilung: **09.09.92 Patentblatt 92/37**

(84) Benannte Vertragsstaaten: **BE CH DE ES FR GB IT LI NL SE**

(56) Entgegenhaltungen:
**DE-A- 3 434 447**

**CHEMICAL ABSTRACTS, Band 107, 1987, Zusammenfassung Nr. 236243k, Columbus, Ohio, US; & JP-A-62 10 041 (SUMITOMO CHEMICAL CO., LTD) 19-01-1987**

(73) Patentinhaber: **BAYER AG**
**W-5090 Leverkusen 1 Bayerwerk(DE)**

(72) Erfinder: **Haug, Michael, Dr.**
**Fahner Weg 5**
**W-5060 Bergisch-Gladbach 2(DE)**
Erfinder: **Andree, Roland, Dr.**
**Schillerstrasse 17**
**W-4018 Langenfeld(DE)**
Erfinder: **Santel, Hans-Joachim, Dr.**
**Gruenstrasse 9a**
**W-5090 Leverkusen 1(DE)**
Erfinder: **Schmidt, Robert R., Dr.**
**Im Waldwinkel 110**
**W-5060 Bergisch-Gladbach 2(DE)**
Erfinder: **Strang, Harry, Dr.**
**Unterdorfstrasse 6a**
**W-4000 Düsseldorf 31(DE)**

EP 0 315 008 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue (6-(Hetero)Aryloxynaphthalin-2-yl-oxy)-alkancarbonsäure-Derivate, Verfahren und neue Zwischenprodukte zu ihrer Herstellung sowie ihre Verwendung als Herbizide.

Es ist bereits bekannt, daß bestimmte Dioxy-benzol-Derivate, wie z. B. α-(4-(2,4-Dichlor-phenoxy)-phenoxy)-propionsäure-methylester (Diclofop-methyl) herbizid wirksam sind (vgl. DE-OS 22 23 894). Desweiteren werden in der DE-OS-34 34 447 und der JP-A-62 10 041 7-(Aryloxy)-2-Naphthyloxy-Alkancarbonsäure derivate, die eine herbizide Wirkung zeigen, beschrieben. Die Wirkung dieser bekannten Verbindungen gegen Unkräter sowie ihre Kulturpflanzen-Verträglichkeit sind jedoch nicht immer zufriedenstellend.

Es wurden nun neue (6-(Hetero)Aryloxy-naphthalin-2-yl-oxy)-alkancarbonsäure-Derivate der allgemeinen Formel (I).

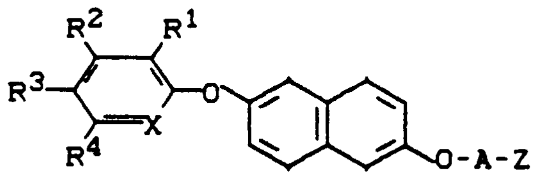

(I)

in welcher

- $R^1$ für Wasserstoff, Halogen, Cyano oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht,
- X für Stickstoff oder die Gruppierung C-$R^5$ steht, worin
  $R^5$ für Wasserstoff oder Halogen steht,
- A für gegebenenfalls verzweigtes Alkandiyl steht und
- Z für Cyano oder die Gruppierung -CO-Y steht, worin
  Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin
  $R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall-oder Erdalkalimetall-äquivalent steht oder für die Gruppierung

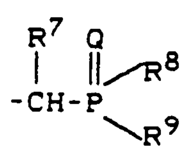

  steht, worin
  $R^7$ für Wasserstoff, Alkyl, Aryl, Furyl, Thienyl oder Pyridyl steht,
  $R^8$ für Alkyl oder Alkoxy steht,
  $R^9$ für Alkoxy steht und
  Q für Sauerstoff oder Schwefel steht,
  oder
  $R^6$ weiterhin für die Gruppierung $-(CH_2)_n-R^{10}$ steht, worin
  $R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht,
gefunden.

Soweit die neuen (6-(Hetero)Aryloxy-naphthalin-2-yl-oxy)-alkancarbonsäure-Derivate asymmetrische Kohlenstoffatome enthalten, betrifft die Erfindung sowohl die einzelnen möglichen Isomeren als auch Gemische dieser Isomeren.

Weiter wurde gefunden, daß man die neuen (6-(Hetero)-Aryloxy-naphthalin-2-yl-oxy)-alkancarbonsäure-Derivate der Formel (I) erhält, wenn man

(a) 6-(Hetero)Aryloxy-2-naphthole der allgemeinen Formel (II)

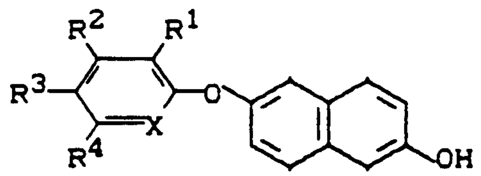

(II)

in welcher
$R^1$, $R^2$, $R^3$, $R^4$ und X die oben angegebenen Bedeutungen haben,
mit Carbonsäurederivaten der allgemeinen Formel (III)

$Z^1$ - A - Z (III)

in welcher
A und Z die oben angegebenen Bedeutungen haben und
$Z^1$ für eine nucleophile Abgangsgruppe steht,
gegebenenfalls in Gegenwart eines Säureakzeptors und gegebenenfalls in Gegenwart eines Verdünnungsmittels umsetzt, oder

(b) Halogen-(hetero)aryl-Verbindungen der allgemeinen Formel (IV)

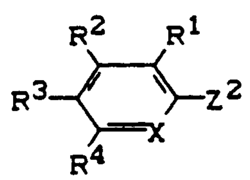

(IV)

in welcher
$R^1$, $R^2$, $R^3$, $R^4$ und X die oben angegebenen Bedeutungen haben und
$Z^2$ für Halogen steht,
mit (6-Hydroxy-naphthalin-2-yl-oxy)-alkancarbonsäure-Derivaten der allgemeinen Formel (V)

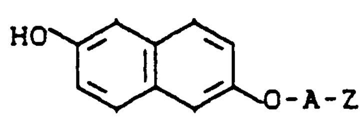

(V)

in welcher
A und Z die oben angegebenen Bedeutungen haben,
gegebenenfalls in Gegenwart eines Säureakzeptors und gegebenenfalls in Gegenwart eines Verdünnungsmittels umsetzt, oder

(c) für den Fall, daß in der Formel (I) Z für die Gruppierung -CO-Y steht, worin Y für Hydroxy steht und

A, $R^1$, $R^2$, $R^3$, $R^4$ und X die oben angegebenen Bedeutungen haben, wenn man Verbindungen der Formel (I), in welcher Z für Cyano oder für die Gruppierung -CO-Y steht, worin Y für Methoxy oder Ethoxy steht und A, $R^1$, $R^2$, $R^3$, $R^4$ und Y die oben angegebenen Bedeutungen haben,
mit einem Alkalihydroxid in Gegenwart eines organischen Lösungsmittels umsetzt und dann - gegebenenfalls nach Einengen - mit einer Mineralsäure ansäuert, oder
(d) für den Fall, daß in der Formel (I) Z für die Gruppierung -CO-Y steht, worin Y für Halogen steht und A, $R^1$, $R^2$, $R^3$, $R^4$ und X die oben angegebenen Bedeutungen haben, wenn man Verbindungen der allgemeinen Formel (I), in welcher Z für die Gruppierung -CO-Y steht, worin Y für Hydroxy steht und A, $R^1$, $R^2$, $R^3$, $R^4$ und X die oben angegebenen Bedeutungen haben, mit einem Halogenierungsmittel gegebenenfalls in Gegenwart eines Verdünnungsmittels und gegebenenfalls in Gegenwart eines Katalysators umsetzt, oder
(e) für den Fall, daß in der Formel (I) Z für die Gruppierung -CO-Y steht, worin Y mit Ausnahme von Halogen die oben angegebene Bedeutung hat und A, $R^1$, $R^2$, $R^3$, $R^4$ und X die oben angegebenen Bedeutungen haben, wenn man Verbindungen der allgemeinen Formel (I), in welcher Z für die Gruppierung -CO-Y steht, worin Y für Halogen steht und A, $R^1$, $R^2$, $R^3$, $R^4$ und X die oben angegebenen Bedeutungen haben,
mit Verbindungen der allgemeinen Formel (VI)

H - Y (VI)

in welcher
Y mit Ausnahme von Halogen die oben angegebene Bedeutung hat,
gegebenenfalls in Gegenwart eines Säureakzeptors und gegebenenfalls in Gegenwart eines Verdünnungsmittels umsetzt, oder
(f) für den Fall, daß in der Formel (I) Z für die Gruppierung -CO-Y steht, worin Y für die Gruppierung -O-$R^6$ steht, worin $R^6$ mit Ausnahme von Ammonium, Alkylammonium, Alkalimetall und Erdalkalimetall die oben angegebene Bedeutung hat und A, $R^1$, $R^2$, $R^3$, $R^4$ und X die oben angegebenen Bedeutungen haben, wenn man Verbindungen der allgemeinen Formel (I), in welcher Z für die Gruppierung -CO-Y steht, worin Y für Hydroxy steht und A, $R^1$, $R^2$, $R^3$, $R^4$ und Y die oben angegebenen Bedeutungen haben,
mit Verbindungen der allgemeinen Formel (VII)

$Z^3$ - $R^{6-1}$ (VII)

in welcher
$R^{6-1}$ mit Ausnahme von Ammonium, Alkylammonium, Alkalimetall und Erdalkalimetall die oben für $R^6$ angegebene Bedeutung hat und
$Z^3$ für Halogen steht,
gegebenenfalls in Gegenwart eines Säureakzeptors und gegebenenfalls in Gegenwart eines Verdünnungsmittels umsetzt, oder
(g) für den Fall, daß in der Formel (I) Z für die Gruppierung -CO-Y steht, worin Y für die Gruppierung -O-$R^6$ steht, worin $R^6$ mit Ausnahme von Ammonium, Alkylammonium, Alkalimetall und Erdalkalimetall die oben angegebene Bedeutung hat, und A, $R^1$, $R^2$, $R^3$, $R^4$ und X die oben angegebenen Bedeutungen haben, wenn man Verbindungen der Formel (I), in welcher Z für Cyano steht und A, $R^1$, $R^2$, $R^3$, $R^4$ die oben angegebenen Bedeutungen haben
mit Hydroxylverbindungen der allgemeinen Formel (VIII)

HO-$R^{6-2}$ (VIII)

in welcher
$R^{6-2}$ für $C_1$-$C_6$-Alkyl, $C_3$-$C_4$-Alkenyl, $C_3$-$C_4$-Alkinyl, $C_1C_4$-Alkoxy-$C_1$-$C_4$-alkyl, $C_1$-$C_4$-Alkylthio-$C_1 = C_4$-alkyl, Phenoxy-$C_1$-$C_3$-alkyl, Phenylthio-$C_1$-$C_3$-alkyl, Benzyloxy-$C_1$-$C_3$-alkyl, Benzylthio-$C_1$-$C_3$-alkyl und $C_1$-$C_4$-Alkoxycarbonyl-$C_1$-$C_2$-alkyl steht,
in Gegenwart einer Mineralsäure und gegebenenfalls in Gegenwart eines Verdünnungsmittels umsetzt.
Schließlich wurde gefunden, daß die neuen (6-(Hetero)-Aryloxy-naphthalin-2-yl-oxy)-alkancarbonsäure-Derivate der allgemeinen Formel (I) starke herbizide Eigenschaften aufweisen.

Überraschenderweise zeigen die erfindungsgemäßen (6-(Hetero)Aryloxy-naphthalin-2-yl-oxy)-alkancarbonsäure-Derivate der Formel (I) bei guter Verträglichkeit in wichtigen Kulturpflanzen gegen Problemunkräuter wesentlich stärkere Wirkung als α-(4-(2,4-Dichlorphenoxy)-phenoxy)-propionsäure-methylester, welches ein strukturell ähnlicher vorbekannter Wirkstoff gleicher Wirkungsrichtung ist.

Die Erfindung betrifft vorzugsweise Verbindungen der Formel (I), in welcher

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder Trifluormethylsulfonyl steht,

$R^4$ für Wasserstoff, Fluor oder Chlor steht,

X für Stickstoff oder die Gruppierung C-$R^5$ steht, worin,

$R^5$ für Wasserstoff, Fluor, Chlor oder Brom steht,

A für gegebenenfalls verzweigtes $C_1$-$C_4$-Alkandiyl steht und

Z für Cyano oder die Gruppierung -CO-Y steht, worin

Y für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^6$ steht, worin

$R^6$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Phenylaminocarbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium-oder Calcium-äquivalent steht,

oder für die Gruppierung

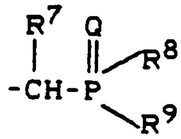

steht, worin

$R^7$ für Wasserstoff, $C_1$-$C_4$-Alkyl, Phenyl, Furyl, Thienyl oder Pyridyl steht,

$R^8$ für $C_1$-$C_4$-Alkyl oder $C_1$-$C_4$-Alkoxy steht,

$R^9$ für $C_1$-$C_4$-Alkoxy steht und

Q für Sauerstoff oder Schwefel steht,

oder

$R^6$ weiterhin für die Gruppierung $-(CH_2)_n-R^{10}$ steht, worin

n für die Zahlen 0, 1 oder 2 steht und

$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,

sowie deren optisch aktive Isomere.

Die Erfindung betrifft insbesondere Verbindungen der Formel (I), in welcher

$R^1$ für Cyano, Fluor oder Chlor steht,

$R^2$ für Wasserstoff, Fluor oder Chlor steht,

$R^3$ für Chlor oder Trifluormethyl steht,

$R^4$ für Wasserstoff, Fluor oder Chlor steht,

X für Stickstoff oder die Gruppierung C-$R^5$ steht, worin

$R^5$ für Wasserstoff, Fluor oder Chlor steht,

A für Methylen ($-CH_2-$), Dimethylen ($-CH_2CH_2-$), Trimethylen $(-CH_2-)_3$ oder Ethyliden

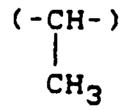

steht und

Z für Cyano oder die Gruppierung -CO-Y steht, worin

Y für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, Diallylamino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung -O-$R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl oder für ein Ammonium-, $C_1$-$C_3$-Alkylammonium-, Natrium-, oder Kalium-äquivalent steht, oder für die Gruppierung

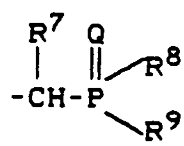

steht, worin

$R^7$ für Wasserstoff, Methyl, Phenyl, Furyl, Thienyl oder Pyridyl steht,

$R^8$ für Methoxy oder Ethoxy steht,

$R^9$ für Methoxy oder Ethoxy steht und

Q für Sauerstoff oder Schwefel steht

oder

$R^6$ weiterhin für die Gruppierung $(-CH_2-)_nR^{10}$ steht, worin

n für die Zahlen 0, 1 oder 2 steht und

$R^{10}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht,

sowie deren optisch aktive Isomere.

Falls die Verbindungen der Formel (I) ein optisch aktives Zentrum haben, sind von den beiden möglichen Isomeren, die R-Enantiomere besonders bevorzugt.

Verwendet man für das erfindungsgemäße Verfahren (a) beispielsweise 6-(3,5-Dichlor-pyridin-2-yl-oxy)-2-naphthol und α-Brom-propionsäure-ethylester als Ausgangsstoffe, so kann der Reaktionsablauf durch das folgende Formelschema wiedergegeben werden:

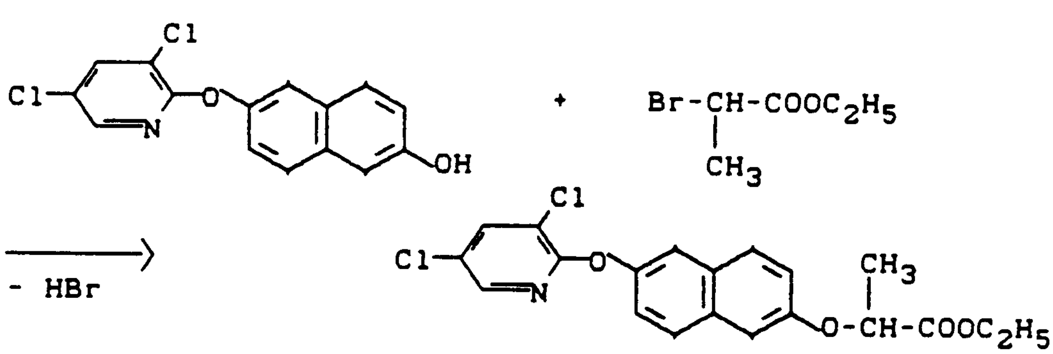


Verwendet man für das erfindungsgemäße Verfahren (b) beispielsweise 3,4,5-Trichlor-benzotrifluorid und (6-Hydroxy-naphthalin-2-yl-oxy)-essigsäure-butylester als Ausgangssstoffe, so kann der Reaktionsablauf durch das folgende Formelschema wiedergegeben werden:

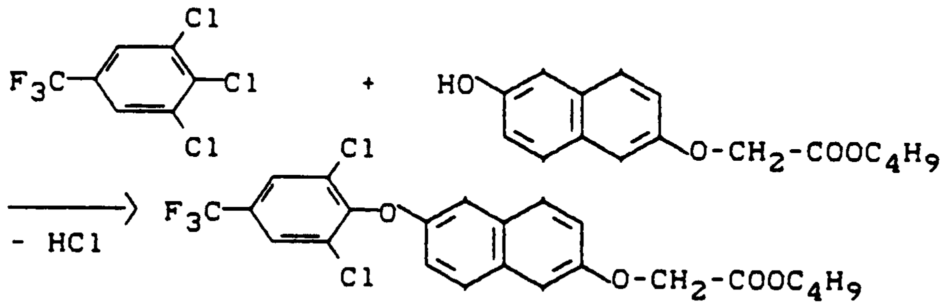


Verwendet man für das erfindungsgemäße Verfahren (c) beispielsweise β-(6-(2-Chlor-4-trifluormethylphenoxy)-naphthalin-2-yl-oxy)-propionsäure-methylester und Natronlauge als Ausgangsstoffe,so kann der Reaktionsablauf durch das folgende Formelschema wiedergegeben werden:

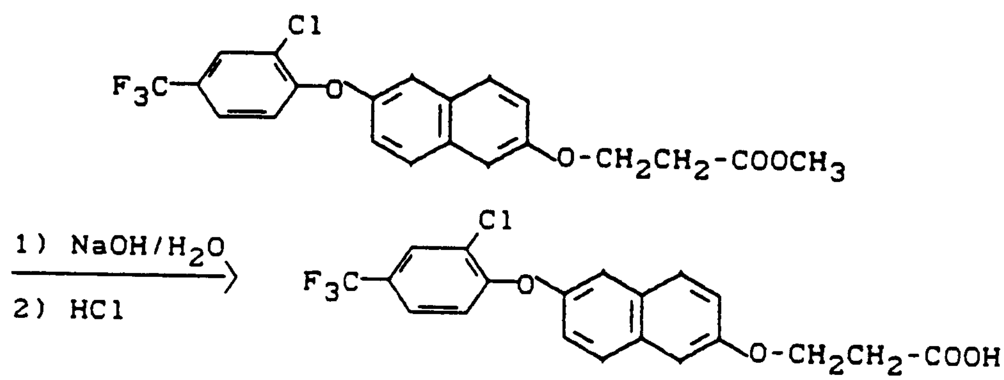


Verwendet man für das erfindungsgemäße Verfahren (d) beispielsweise α-(6-(2,6-Dichlor-3-fluor-4-trifluormethyl-phenoxy)-naphthalin-2-yl-oxy)-propionsäure und Thionylchlorid als Ausgangsstoffe, so kann der Reaktionsablauf durch das folgende Formelschema wiedergegeben werden:

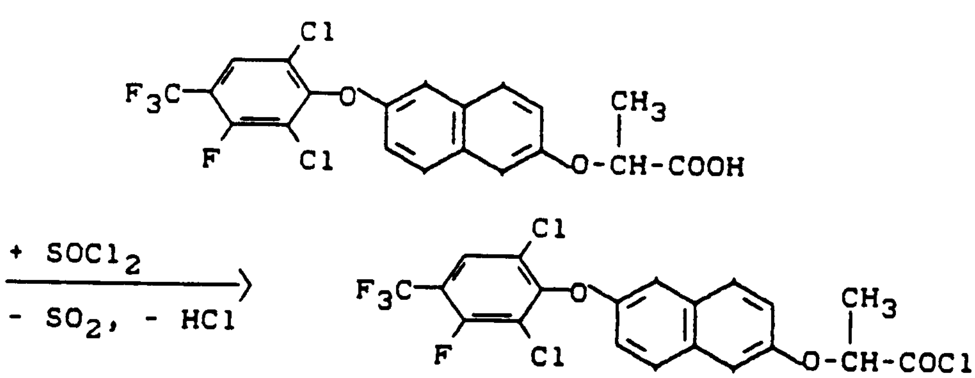


Verwendet man für das erfindungsgemäße Verfahren (e) beispielsweise α-(6-(2-Chlor-6-fluor-4-trifluormethylphenoxy)-naphthalin-2-yl-oxy)-propionsäure-chlorid und Mercaptoessigsäuremethylester als Ausgangsstoffe, so kann der Reaktionsablauf durch das folgende Formelschema wiedergegeben werden:

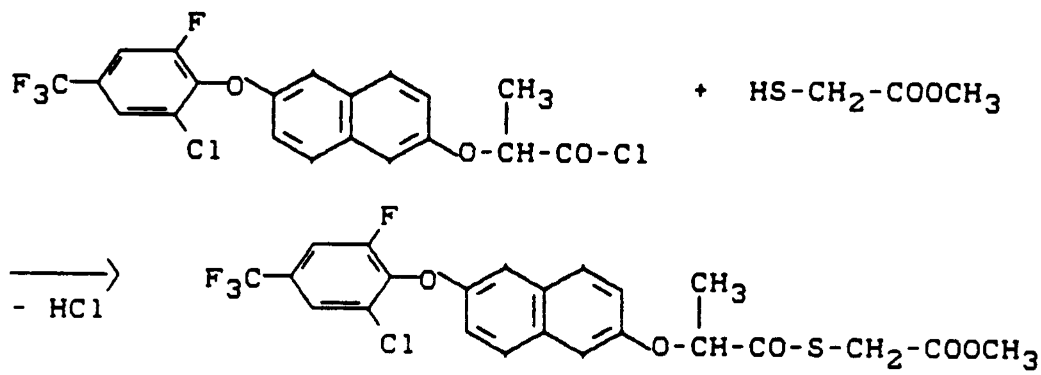


Verwendet man für das erfindungsgemäße Verfahren (f) beispielsweise (6-(2,3,6-Trichlor-4-trifluormethyl-phenoxy)-naphthalin-2-yl-oxy)-essigsäure und Trimethylsilylmethylchlorid als Ausgangsstoffe, so kann der Reaktionsablauf durch das folgende Formelschema wiedergegeben werden:

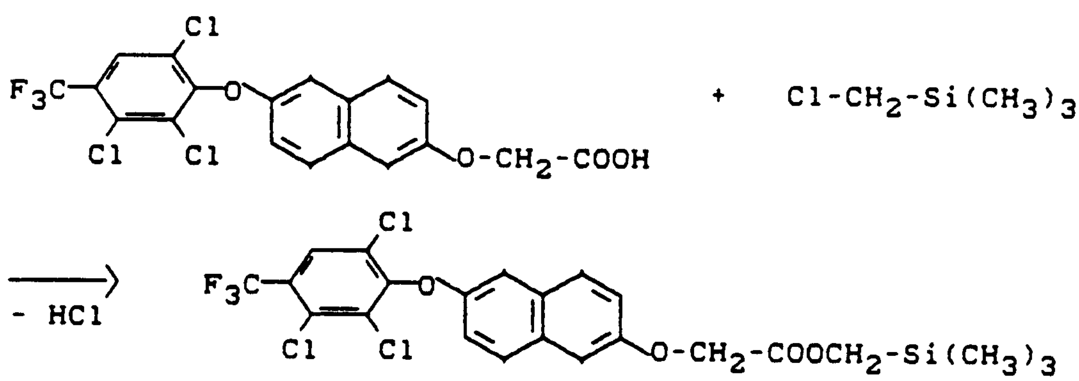


Verwendet man für das erfindungsgemäße Verfahren (g) beispielsweise (6-(2-Chlor-6-fluor-4-trifluormethylphenoxy)-naphthalin-2-yl-oxy)-acetonitril und Methanol als Ausgangsstoffe, so kann der Reaktionsverlauf durch das folgende Formelschema wiedergegeben werden:

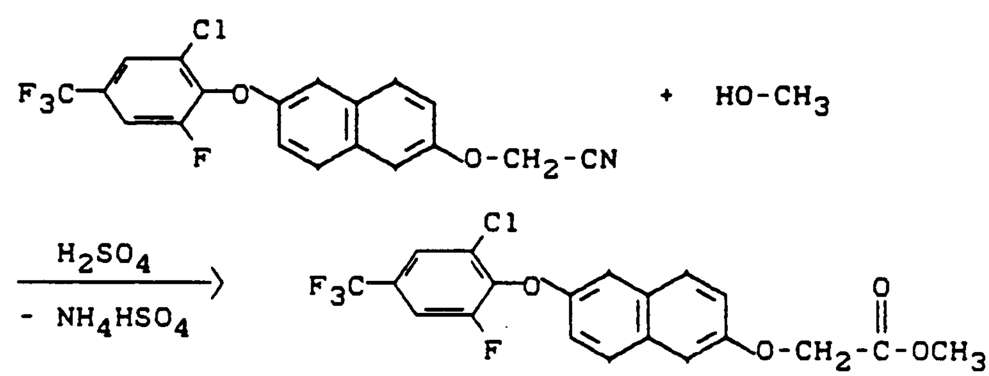


Die beim erfindungsgemäßen Verfahren (a) zur Herstellung von Verbindungen der Formel (I) als Ausgangsstoffe zu verwendenden 6-(Hetero)Aryloxy-2-naphthole sind durch die Formel (II) allgemein definiert.

In Formel (II) haben $R^1$, $R^2$, $R^3$, $R^4$ und X vorzugsweise bzw. insbesondere diegenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden.

Als Beispiele für die Ausgangsstoffe der Formel (II) seien genannt:

6-(4-Trifluormethyl-phenoxy)-2-naphthol, 6-(2-Chlor-4-trifluormethyl-phenoxy)-2-naphthol, 6-(2,6-Dichlor-4-trifluormethyl-phenoxy)-2-naphthol, 6-(2-Chlor-6-fluor-4-trifluormethyl-phenoxy)-2-naphthol, 6-(2,6-Dichlor-3-fluor-4-trifluormethyl-phenoxy)-2-naphthol, 6-(2,3,6-Trichlor-4-trifluormethyl-phenoxy)-2-naphthol und 6-(3,5-Dichlorpyridin-2-yl-oxy)-2-naphthol.

Die Ausgangsstoffe der Formel (II) sind noch nicht aus der Literatur bekannt. Man erhält die Verbindungen der Formel (II), wenn man entsprechende Halogen(hetero)aryl-Verbindungen der allgemeinen Formel (IV)

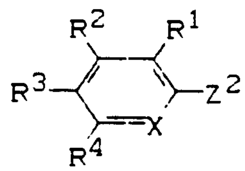

(IV)

in welcher

$R^1$, $R^2$, $R^3$, $R^4$, X und $Z^2$ die oben angegebenen Bedeutungen haben,

mit 2,6-Dihydroxynaphthalin in Gegenwart eines Säureakzeptors, wie z. B. Natriumhydroxid oder Kaliumhydroxid, und in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen 20 °C und 150 °C umsetzt und nach üblichen Methoden aufarbeitet.

Die Halogen-(hetero)aryl-Verbindungen sind durch die Formel (IV) allgemein definiert. In Formel (IV) haben $R^1$, $R^2$, $R^3$, $R^4$ und X vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden und $Z^2$ steht vorzugsweise für Chlor oder Fluor.

Als Beispiele für die Halogen-(hetero)aryl-Verbindungen der Formel (IV) seien genannt:

4-Chlor-benzotrifluorid, 3,4-Dichlor-benzotrifluorid, 3,4,5-Trichlor-benzotrifluorid, 3,4-Dichlor-5-fluorbenzotrifluorid, 2,3,4,5-Tetrachlor-benzotrifluorid, 3,5-Dichlor-2,4-difluor-benzotrifluorid, 3-Chlor-4,5-difluor-benzotrifluorid und 2,3,5-Trichlor-pyridin.

Die Verbindungen der Formel (IV) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; FR-A 2 538 380 (Chem. Abstracts 102 (1985), 61914x)).

Die beim erfindungsgemäßen Verfahren (a) weiter als Ausgangsstoffe zu verwendenden Carbonsäurederivate sind durch die Formel (III) allgemein definiert. In Formel (III) haben A und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt angegeben wurden und $Z^1$ steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methylphenylsulfonyloxy.

Als Beispiele für die Verbindungen der Formel (III) seien genannt:

Chloracetonitril, Bromacetonitril, β-Brom-propionitril, β-Chlor-propionitril, α-Chlor-, α-Brom- und α-Iod-propionsäure-methylester, -ethylester, -propylester, -isopropylester, -butylester, -isobutylester und -sec-butylester, β-Chlor-, β-Brom- und β-Iod-propionsäure-methylester, -ethylester, -propylester, -isopropylester, -butylester, -isobutylester und -sec-butylester, Chlor-, Brom- und Iod-essigsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methyl-phenylsulfonyloxy)-propionsäure-methylester, -ethylester, -propylester, -butylester, -isopropylester, -isobutylester und -sec-butylester.

Die Ausgangsstoffe der Formel (III) sind bekannt und/oder können nach an sich bekannten Verfahren hergestellt werden (vgl. DE-OS 27 58 002, DE-OS 28 54 542).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und 0-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahen (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man je Mol 6-(Hetero)Aryloxy-2-naphthol der Formel (II) im allgemeinen zwischen 0,5 und 1,2 Mol, vorzugsweise zwischen 0,7 und 1,0 Mol Carbonsäurederivat der Formel (III) ein. Die Reaktionskomponenten werden im allgemeinen bei Raumtemperatur oder unter leichtem Kühlen zusammengegeben und dann - gegebenenfalls bei erhöhter Temperatur - bis zum Ende der Umsetzung gerührt.

Die Aufarbeitung kann auf übliche Weise durchgeführt werden. Beispielsweise wird das Reaktionsgemisch mit einer Säure, wie z. B. Salzsäure oder Schwefelsäure und Wasser, sowie einem mit Wasser praktisch nicht mischbaren organischen Lösungsmittel, wie z. B. Methylenchlorid oder Toluol, verrührt oder geschüttelt, die organische Phase abgetrennt, mit Wasser gewaschen, getrocknet und filtriert. Das nach Einengen des Filtrats im Rückstand verbleibende Produkt der Formel (I) kann auf übliche Weise, beispielsweise durch Säulenchromatographie, gereinigt werden.

Die beim erfindungsgemäßen Verfahren (b) als Ausgangsstoffe zu verwendenden Halogen-heteroaryl-Verbindungen wurden bereits oben beschrieben.

Die beim erfindungsgemäßen Verfahren (b) weiter als Ausgangsstoffe zu verwendenden (6-Hydroxy-naphthalin-2-yl-oxy)-alkancarbonsäure-Derivate sind durch die Formel (V) allgemein definiert. In Formel (V) haben A und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt angegeben wurden.

Als Beispiele für die Ausgangsstoffe der Formel (V) seien genannt:

α-(6-Hydroxy-naphthalin-2-yl-oxy)-propionsäure-nitril, -methylester, -ethylester, -propylester, -isopropylester, -butylester, -isobutylester und -sec-butylester,

β-(6-Hydroxy-napthalin-2-yl-oxy)-propionsäure-nitril, -methylester, -ethylester, -propylester, -isopropylester, -butylester, -isobutylester und -sec-butylester, (6-Hydroxy-naphthalin-2-yl-oxy)-essigsäure-nitril, -methylester, -ethylester, -propylester, -isopropylester, -butylester, -isobutylester und -sec-butylester.

Die Ausgangsstoffe der Formel (V) sind bekannt und/oder können nach an sich bekannten Verfahren hergestellt werden (vgl. US-P 3 740 437).

Verfahren (b) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden. Aprotisch polare organische Lösungsmittel, wie z. B. Aceton, Acetonitril, Methylethylketon, Propionitril, Dimethylformamid, Dimethylacetamid, Dimethylsulfoxid, Sulfolan und N-Methylpyrrolidon werden besonders bevorzugt.

Verfahren (b) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man je Mol Halogen-(hetero)aryl-Verbindung der Formel (IV) im allgemeinen zwischen 0,5 und 2 Mol vorzugsweise zwischen 0,7 und 1,5

Mol, (6-Hydroxy-naphthalin-2-yl-oxy)-alkancarbonsäure-Derivat der Formel (V) ein.

Umsetzung und Aufarbeitung können wie oben für Verfahren (a) beschrieben durchgeführt werden.

Die beim erfindungsgemäßen Verfahren (c) als Ausgangsstoffe zu verwendenden Verbindungen sind durch die Formel (I) mit der Maßgabe, daß Z für Cyano oder die Gruppierung -CO-Y steht, worin Y für Methoxy oder Ethoxy steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (c) seien genannt:

α-(6-(3,5-Dichlor-pyridin-2-yl-oxy)-, α-(6-(4-Trifluormethyl-phenoxy)-, α-(6-(2-Chlor-4-trifluormethyl-phenoxy)-, α-(6-(2,6-Dichlor-4-trifluormethyl-phenoxy)-, α-(6-(2-Chlor-6-fluor-4-trifluormethyl-phenoxy)-, α-(6-(2,3,6-Trichlor-4-trifluormethyl-phenoxy)- und α-(6-(2,6-Dichlor-3-fluor-4-trifluormethyl-phenoxy)-naphthalin-2-yl-oxy-)-propionsäure-nitril, -methylester und -ethylester;

β-(6-(3,5-Dichlor-pyridin-2-yl-oxy)-, β-(6-(4-Trifluormethyl-phenoxy)-, β-(6-(2-Chlor-4-trifluormethyl-phenoxy)-, β-(6-(2,6-Dichlor-4-trifluormethyl-phenoxy)-, β-(6-(2-Chlor-6-fluor-4-trifluormethyl-phenoxy)-, β-(6-(2,3,6-Trichlor-4-trifluormethyl-phenoxy)- und β-(6-(2,6-Dichlor-3-fluor-4-trifluormethyl-phenoxy)-naphthalin-2-yl-oxy)-propionsäure-nitril, -methylester und -ethylester;

(6-(3,5-Dichlor-pyridin-2-yl-oxy)-, (6-(4-Trifluormethyl-phenoxy)-, (6-(2-Chlor-4-trifluoromethyl-phenoxy)-, (6-(2,6-Dichlor-4-trifluormethyl-phenoxy)-, (6-(2-Chlor-6-fluor-4-trifluormethyl-phenoxy)-, (6-(2,3,6-Trichlor-4-trifluormethyl-phenoxy)- und (6-(2,6-Dichlor-3-fluor-4-trifluormethyl-phenoxy)-naphthalin-2-yl-oxy)-essigsäure-nitril, -methylester und -ethylester.

Die oben beschriebenen Ausgangsstoffe der Formel (I) für Verfahren (c) sind erfindungsgemäße, neue Verbindungen; sie können nach dem erfindungsgemäßen Verfahren (a) oder (b) hergestellt werden.

Verfahren (c) wird unter Verwendung von Alkalihydroxiden durchgeführt. Als Beispiel hierfür seien Lithiumhydroxid, Natriumhydroxid und Kaliumhydroxid genannt. Vorzugsweise wird Natriumhydroxid verwendet.

Verfahren (c) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol eingesetzt.

Zum Ansäuern werden bei Verfahren (c) die üblichen Mineralsäuren, wie z. B. Salzsäure oder Schwefelsäure, verwendet.

Die Reaktionstemperaturen können bei dem erfindungsgemäßen Verfahren (c) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung von Verfahren (c) werden je Mol Ausgangsverbindung der Formel (I) im allgemeinen zwischen 0,1 und 10 Mol, vorzugsweise zwischen 0,5 und 2 Mol, Alkalihydroxid eingesetzt. Die Reaktionskomponenten werden im allgemeinen bei Raumtemperatur zusammengegeben und das Reaktionsgemisch wird gegegenenfalls bei erhöhter Temperatur, bis zum Reaktionsende gerührt. Das - gegebenenfalls nach Einengen, Abkühlen und Ansäuern - kristallin anfallende Reaktionsprodukt kann durch Absaugen isoliert werden.

Die beim erfindungsgemäßen Verfahren (d) als Ausgangsstoffe zu verwendenden Verbindungen sind durch die Formel (I) mit der Maßgabe, daß Z für die Gruppierung -CO-Y steht, worin Y für Hydroxy steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (d) seien genannt:

α-(6-(3,5-Dichlor-pyridin-2-yl-oxy)-, α-(6-(4-Trifluormethyl-phenoxy)-, α-(6-(2-Chlor-4-trifluormethyl-phenoxy)-, α-(6-(2,6-Dichlor-4-trifluormethyl-phenoxy)-, α-(6-(2-Chlor-6-fluor-4-trifluormethyl-phenoxy)-, α-(6-(2,3,6-Trichlor-4-trifluormethyl-phenoxy)- und α-(6-(2,6-Dichlor-3-fluor-4-trifluormethyl-phenoxy)-naphthalin-2-yl-oxy)-propionsäure;

β-(6-(3,5-Dichlor-pyridin-2-yl-oxy)-, β-(6-(4-Trifluormethyl-phenoxy)-, β-(6-(2-Chlor-4-trifluormethyl-phenoxy)-, β-(6-(2,6-Dichlor-4-trifluormethyl-phenoxy)-, β-(6-(2-Chlor-6-fluor-4-trifluormethyl-phenoxy)-, β-(6-(2,3,6-Trichlor-4-trifluormethyl-phenoxy)- und β-(6-(2,6-Dichlor-3-fluor-4-trifluormethyl-phenoxy)-naphthalin-2-yl-oxy)-propionsäure;

(6-(3,5-Dichlor-pyridin-2-yl-oxy)-, (6-(4-Trifluormethyl-phenoxy)-, (6-(2-Chlor-4-trifluormethyl-phenoxy)-, (6-(2,6-Dichlor-4-trifluormethyl-phenoxy)-, (6-(2-Chlor-6-fluor-4-trifluormethyl-phenoxy)-, (6-(2,3,6-Trichlor-4-trifluormethyl-phenoxy)- und (6-(2,6-Dichlor-3-fluor-4-trifluormethyl-phenoxy)-naphthalin-2-yl-oxy)-essigsäure.

Die oben beschriebenen Ausgangsstoffe der Formel (I) für Verfahren (d) sind erfindungsgemäße, neue Verbindungen; sie können nach dem erfindungsgemäßen Verfahren (c) hergestellt werden.

Verfahren (d) wird unter Verwendung eines Halogenierungsmittels durchgeführt. Es können die üblichen Mittel für die Umsetzung von Carbonsäuren zur Carbonsäurehalogeniden eingesetzt werden. Als Beispiele hierfür seien Phosgen, Thionylchlorid, Phosphorylchlorid und Benzotrichlorid genannt. Vorzugsweise wird Thionylchlorid als Halogenierungsmittel verwendet.

Verfahren (d) wird gegebenenfalls in Gegenwart eines Katalysators durchführt. Es können die für die Herstellung von Säurechloriden aus Säuren üblichen Katalysatoren, wie z. B. Pyridin oder Dimethylformamid verwendet werden.

Verfahren (d) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (d) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann auf übliche Weise gereinigt werden, aber auch ohne weitere Reinigung für Folgeumsetzungen eingesetzt werden.

Die beim erfindungsgemäßen Verfahren (e) als Ausgangsstoffe zu verwendenden Verbindungen sind durch die Formel (I) mit der Maßgabe, daß Z für die Gruppierung -CO-Y steht, worin Y für Halogen steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw, als insbesondere bevorzugt angegeben wurden und Y steht vorzugsweise für Fluor, Chlor oder Brom, insbesondere für Chlor.

Als Beispiele für die Ausgangsstoffe zu Verfahren (e) seien genannt:

α-(6-(3,5-Dichlor-pyridin-2-yl-oxy)-, α-(6-(4-Trifluormethyl-phenoxy)-, α-(6-(2-Chlor-4-trifluormethyl-phenoxy)-, α-(6-(2,6-Dichlor-4-trifluormethyl-phenoxy)-, α-(6-(2-Chlor-6-fluor-4-trifluormethyl-phenoxy)-, α-(6-(2,3,6-Trichlor-4-trifluormethyl-phenoxy)- und α-(6-(2,6-Dichlor-3-fluor-4-trifluormethyl-phenoxy)-naphthalin-2-yl-oxy)-propionsäure-chlorid;

β-(6-(3,5-Dichlor-pyridin-2-yl-oxy)-, β-(6-(4-Trifluormethyl-phenoxy)-, β-(6-(2-Chlor-4-trifluormethyl-phenoxy)-, β-(6-(2,6-Dichlor-4-trifluormethyl-phenoxy)-, β-(6-(2-Chlor-6-fluor-4-trifluormethyl-phenoxy)-, β-(6-(2,3,6-Trichlor-4-trifluormethyl-phenoxy)- und β-(6-(2,6-Dichlor-4-fluor-4-trifluormethyl-phenoxy)-naphtha lin-2-yl-oxy)-propionsäure-chlorid;

(6-(3,5-Dichlor-pyridin-2-yl-oxy)-, (6-(4-Trifluormethyl-phenoxy)-, (6-(2-Chlor-4-trifluormethyl-phenoxy)-, (6-(2,6-Dichlor-4-trifluormethyl-phenoxy)-, (6-(2-Chlor-6-fluor-4-trifluormethyl-phenoxy)-, (6-(2,3,6-Trichlor-4-trifluormethyl-phenoxy)- und (6-(2,6-Dichlor-3-fluor-4-trifluormethyl-phenoxy)-naphthalin-2-yl-oxy)-essigsäure-chlorid.

Die oben beschriebenen Ausgangsstoffe der Formel (I) für Verfahren (e) sind erfindungsgemäße, neue Verbindungen; sie können nach dem erfindungsgemäßen Verfahren (d) hergestellt werden.

Die beim erfindungsgemäßen Verfahren (e) weiter als Ausgangsstoffe einzusetzenden Verbindungen sind durch die Formel (VI) allgemein definiert. In Formel (VI) hat Y mit Ausnahme von Halogen vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde.

Als Beispiele für die Ausgangsstoffe der Formel (VI) seien genannt:

Methylamin, Ethylamin, Propylamin, Isopropylamin, Anilin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxy-ethanol, 3-Benzyloxy-propanol, 2-Benzylthio-ethanol, Hydroxymethanphosphonsäure-diethylester und -dimethylester, 1-Hydroxy-ethan-phosphonsäure-dimethylester und -diethylester, 1-Hydroxy-1-phenyl-methanphosphonsäure-dimethylester und -diethylester, Acetonoxim, 3-Hydroxyfuran, Furfurylalkohol, Perhydrofurfurylalkohol, Milchsäure-methylester und -ethylester und Glycolsäure-methylester und -ethylester.

Diese Verbindungen sind bekannte Synthesechemikalien.

Verfahren (e) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden.

Verfahren (e) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (e) in einem größeren Bereich variiert werden. Im allgemeinen arbeitet man bei Temperaturen zwischen -20 °C und +100 °C, vorzugsweise bei Temperaturen zwischen 0 °C und 50 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (e) jeweils nach üblichen Methoden. Beispielsweise wird das Reaktionsgemisch - gegebenenfalls nach Einengen - mit Wasser verdünnt und das gewünschte Reaktionsprodukt wird mit einem mit Wasser praktisch nicht mischbaren organischen Lösungsmittel, z. B. Methylenchlorid, Chloroform, Diethylether, Toluol oder Xylol, extrahiert. Die organische Extraktionslösung wird mit Wasser gewaschen, mit einem üblichen Trockenmittel, wie z. B. Natriumsulfat, getrocknet und filtriert. Nach dem Einengen des Filtrats erhält man die Verbindungen der Formel (I) als Rohprodukte, welche auf übliche Weise, z. B. chromatographisch und/oder durch Umkristallisation gereinigt werden können.

Die beim erfindungsgemäßen Verfahren (f) als Ausgangsstoffe zu verwendenden Verbindungen sind durch die Formel (I) mit der Maßgabe, daß Z für die Gruppierung -CO-Y steht, worin Y für Hydroxy steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (f) seien genannt:

α-(6-(3,5-Dichlor-pyridin-2-yl-oxy)-, α-(6-(4-Trifluormethyl-phenoxy)-, α-(6-(2-Chlor-4-trifluormethyl-phenoxy)-, α-(6-(2,6-Dichlor-4-trifluormethyl-phenoxy)-, α-(6-(2-Chlor-6-fluor-4-trifluormethyl-phenoxy)-, α-(6-(2,3,6-Trichlor-4-trifluormethyl-phenoxy)- und α-(6-(2,6-Dichlor-3-fluor-4-trifluormethyl-phenoxy)-naphthalin-2-yl-oxy)-propionsäure;

β-(6-(3,5-Dichlor-pyridin-2-yl-oxy)-, β-(6-(4-Trifluormethyl-phenoxy)-, β-(4-(2-Chlor-4-trifluormethyl-phenoxy)-, β-(6-(2,6-Dichlor-4-trifluormethyl-phenoxy)-, β-(6-(2-Chlor-6-fluor-4-trifluormethyl-phenoxy)-, β-(6-(2,3,6-Trichlor-4-trifluormethyl-phenoxy)- und β-(6-(2,6-Dichlor-3-fluor-4-trifluormethyl-phenoxy)-naphthalin-2-yl-oxy)-propionsäure;

(6-(3,5-Dichlor-pyridin-2-yl-oxy)-, (6-(4-Trifluormethyl-phenoxy)-, (6-(2-Chlor-4-trifluormethyl-phenoxy)-, (6-(2,6-Dichlor-4-trifluormethyl-phenoxy)-, (6-(2-Chlor-6-fluor-4-trifluormethyl-phenoxy)-, (6-(2,3,6-Trichlor-4-trifluormethyl-phenoxy)-und (6-(2,6-Dichlor-3-fluor-4-trifluormethyl-phenoxy)-naphthalin-2-yl-oxy)-essigsäure.

Die oben beschriebenen Ausgangsstoffe der Formel (I) für Verfahren (f) sind erfindungsgemäße, neue Verbindungen; sie können nach dem erfindungsgemäßen Verfahren (c) hergestellt werden.

Die beim erfindungsgemäßen Verfahren (f) weiter als Ausgangsstoffe einzusetzenden Verbindungen sind durch die Formel (VII) allgemein definiert. In Formel (VII) stehen vorzugsweise $R^{6-1}$ für $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxycarbonyl-$C_1$-$C_2$-alkyl und $Z^3$ für Chlor, Brom oder Iod.

Trimethylsilylmethylchlorid wird als Ausgangsverbindung der Formel (VII) für Verfahren (f) besonders bevorzugt.

Die Ausgangstoffe der Formel (VII) sind bekannte Synthesechemikalien.

Verfahren (f) wird vorzugsweise unter Verwendung eines Vedünnungsmittels durchgeführt. Es kommen vor allem diejenigen Verdünnungsmittel in Betracht, die bereits bei der Beschreibung des erfindungsgemäßen Verfahrens (a) genannt wurden. Aceton, Acetonitril und Dimethylformamid werden besonders bevorzugt.

Verfahren (f) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden. 1,8-Diazabicyclo-[5.4.0]-undec-7-en (DBU) wird besonders bevorzugt.

Die Reaktionstemperaturen können bei dem erfindungsgemäßen Verfahren (f) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung von Verfahren (f) setzt man je Mol Ausgangsverbindung der Formel (I) im allgemeinen zwischen 1 und 3 Mol, vorzugsweise zwischen 1,1 und 2,5 Mol Ausgangsverbindung der Formel (VII) ein.

Umsetzung und Aufarbeitung können wie oben für Verfahren (a) beschrieben durchgeführt werden.

Die beim erfindungsgemäßen Verfahren (g) als Ausgangsstoffe zu verwendenden Verbindungen sind durch die Formel (I) mit der Maßgabe, daß Z für Cyano steht, allgemein definiert. In diesem Fall haben A, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (g) seien genannt:
α-(6-(3,5-Dichlor-pyridin-2-yl-oxy)-, α-(6-(-4-Trifluormethyl-phenoxy)-, α-(6-(2-Chlor-4-trifluormethyl-phenoxy)-, α-(6-(2,6-Dichlor-4-trifluormethyl-phenoxy)-, α-(6-(2-Chlor-6-fluor-4-trifluormethyl-phenoxy)-, α-(6-(2,3,6-Trichlor-4-trifluormethyl-phenoxy)- und α-(6-(2,6-Dichlor-3-fluor-4-trifluormethyl-phenoxy)-naphthalin-2-yl-oxy)-propionsäurenitril;
β-(6-(3,5-Dichlor-pyridin-2-yl-oxy)-, β-(6-(4-Trifluormethyl-phenoxy)-, β-(6-(2-Chlor-4-trifluormethyl-phenoxy)-, β-(6-(2,6-Dichlor-4-trifluormethyl-phenoxy)-, β-(6-(2-Chlor-6-fluor-4-trifluormethyl-phenoxy)-, β-(6-(2,3,6-Trichlor-4-trifluormethyl-phenoxy)- und β-(6-(2,6-Dichlor-3-fluor-4-trifluormethyl-phenoxy)-naphthalin-2-yl-oxy)-propionsäurenitril;
(6-(3,5-Dichlor-pyridin-2-yl-oxy)-, (6-(4-Trifluormethyl-phenoxy)-, (6-(2-Chlor-4-trifluormethyl-phenoxy)-, (6-(2,6-Dichlor-4-trifluormethyl-phenoxy)-, (6-(2-Chlor-6-fluor-4-trifluormethyl-phenoxy)-, (6-(2,3,6-Trichlor-4-trifluormethyl-phenoxy)- und (6-(2,6-Dichlor-3-fluor-4-trifluormethyl-phenoxy)-naphthalin-2-yl-oxy)-essigsäurenitril.

Die oben beschriebenen Ausgangsstoffe der Formel (I) für Verfahren (g) sind erfindungsgemäße, neue Verbindungen; sie können nach den erfindungsgemäßen Verfahren (a) oder (b) herstellt werden.

Die beim erfindungsgemäßen Verfahren (g) weiter als Ausgangsstoffe einzusetzenden Verbindungen sind durch die Formel (VIII) allgemein definiert. In Formel (VIII) steht $R^{6-2}$ vorzugsweise für $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Phenylthio-$C_1$-$C_3$-alkyl, Benzyloxy-$C_1$-$C_3$-alkyl, Benzylthio-$C_1$-$C_3$-alkyl und $C_1$-$C_4$-Alkoxycarbonyl-$C_1$-$C_2$-alkyl.

Insbesondere steht in Formel (VIII) $R^{6-2}$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, Benzyloxy-$C_1$-$C_3$-alkyl und Benzylthio-$C_1$-$C_3$-alkyl.

Als Beispiele für die Ausgangsstoffe der Formel (VIII) seien genannt:
Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxy-ethanol, 3-Benzyloxy-propanol und 2-Benzylthio-ethanol.

Die Ausgangsstoffe der Formel (VIII) sind bekannte Synthesechemikalien.

Verfahren (g) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden.

Verfahren (g) wird in Gegenwart einer Mineralsäure durchgeführt. Als Mineralsäure werden vorzugsweise Salzsäure oder Schwefelsäure eingesetzt.

Die Reaktionstemperaturen können bei dem erfindungsgemäßen Verfahren (g) in einem größeren Bereich variiert werden. Im allgemeinen arbeitet man bei Temperaturen zwischen 0 °C und 150 °C, vorzugsweise bei Temperaturen zwischen 20 °C und 120 °C.

Das erfindungsgemäße Verfahren wird im allgemeinen bei Normaldruck durchgeführt.

Zur Durchführung von Verfahren (g) setzt man je Mol Ausgangsverbindung der Formel (I) im allgemeinen zwischen 1 und 10 Mol, vorzugsweise zwischen 1,1 und 4 Mol Ausgangsverbindung der Formel (VIII) und der Mineralsäure ein. Die Reaktionskomponenten werden im allgemeinen bei Raumtemperatur oder unter leichtem Kühlen zusammengegeben und dann - gegebenenfalls bei erhöhter Temperatur - bis zum Ende der Umsetzung gerührt. Die Aufarbeitung kann in üblicher Weise durchgeführt werden. Beispielsweise wird das Reaktionsgemisch auf Wasser gegeben sowie mit einem mit Wasser praktisch nicht mischbaren organischen Lösungsmittel, wie z. B. Methylenchlorid oder Toluol, verrührt oder geschüttelt, die organische Phase abgetrennt, mit Wasser gewaschen, getrocknet und filtriert. Das nach Einengen des Filtrats im

Rückstand verbleibende Produkt kann auf übliche Weise gereinigt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung dikotyler Unkräuter, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate

aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (Amethydione) oder N-(2-Benzthiazolyl)-N,N′-dimethyl-harnstoff (Metabenzthiazuron) zur Unkrautbekämpfung in Getreide; 4-Amino-3-methyl-6-phenyl-1,2,4-triazin-5(4H)-on (Metamitron) zur Unkrautbekämpfung in Zuckerrüben und 4-Amino-6-(1,1-dimethylethyl)-3-methylthio-1,2,4-triazin-5(4H)-on (Metribuzin) zur Unkrautbekämpfung in Sojabohnen, in Frage, ferner auch 2,4-Dichlorphenoxyessigsäure (2,4-D); 4-(2,4-Dichlorphenoxy)-buttersäure (2,4-DB); 2,4-Dichlorphenoxy-propionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-Chlor-4-ethylamino-6-(3-cyanopropylamino)-1,3,5-triazin (CYANAZIN); 3,6-Dichlor-2-pyridincarbonsäure (CLOPYRALID); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); 2-[4-(3,5-Dichlorpyrid-2-yloxy)-phenoxy]-propionsäure-(trimethylsilylmethylester); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yl-oxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) und 3,5,6-Trichlor-2-pyridyloxyessigsäure (TRICLOPYR). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

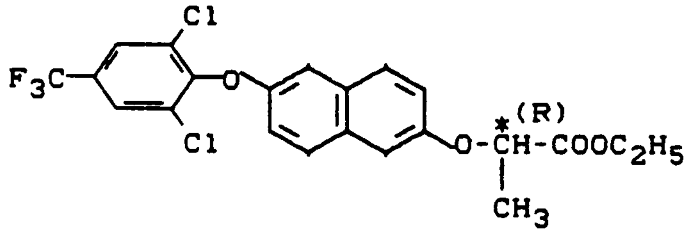

Eine Mischung aus 16,8 g (0,045 Mol) 6-(2,6-Dichlor-4-trifluormethyl-phenoxy)-2-naphthol, 13,6 g (0,050 Mol) (S)-α-(4-Methyl-phenylsulfonyloxy)-propionsäure-ethylester, 6,3 g Kaliumcarbonat und 150 ml Acetonitril wird 20 Stunden unter Rückfluß zum Sieden erhitzt. Nach dem Abkühlen auf ca. 20 °C wird das Reaktionsgemisch mit Wasser auf etwa das doppelte Volumen verdünnt, mit 1N-Salzsäure angesäuert und mit Toluol geschüttelt. Die organische Phase wird abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 5,9 g (28 % der Theorie) (R)-α-(6-(2,6-Dichlor-4-trifluormethyl-phenoxy)-naphthalin-2-yl-oxy)-propionsäure-ethylester. Fp. : 118 °C - 120 °C.

Analog Beispiel 1 und entsprechend der allgemeinen Beschreibung der erfindungsgemäßen Herstellungsverfahren können die in der nachstehenden Tabelle 1 aufgeführten Verbindungen der Formel (I) hergestellt werden:

Tabelle 1: Beispiele für die Verbindungen der Formel (I)

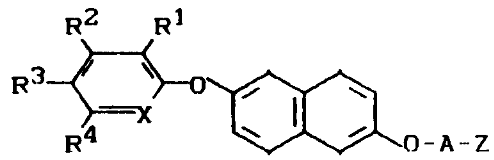

(I)

| Beisp.-Nr. | $R^1$ | $R^2$ | $R^3$ | $R^4$ | X | A | Z | Physikalische Daten |
|---|---|---|---|---|---|---|---|---|
| 2 | H | H | $CF_3$ | H | CH | $-CH-$<br>\|<br>$CH_3$ | $COOC_2H_5$ | $n_D^{20} = 1,5598$ |
| 3 | Cl | H | $CF_3$ | H | CH | $-CH-$<br>\|<br>$CH_3$ | $COOC_2H_5$ | Fp. : 84 °C |
| 4 | Cl | H | $CF_3$ | H | C-F | $-CH-$<br>\|<br>$CH_3$ | $COOC_2H_5$ | Fp. : 53 °C |
| 5 | Cl | Cl | $CF_3$ | H | C-Cl | $-CH-$<br>\|<br>$CH_3$ | $COOC_4H_9$ | |
| 6 | Cl | F | $CF_3$ | H | C-Cl | $-CH-$<br>\|<br>$CH_3$ | $COOCH(CH_3)_2$ | |
| 7 | Cl | H | $CF_3$ | H | C-Cl | $-CH_2-$ | $COOC_4H_9$ | |
| 8 | Cl | H | $CF_3$ | H | C-Cl | $-CH_2CH_2-$ | $COOCH_3$ | |

Tabelle 1 - Fortsetzung

| Beisp.-Nr. | $R^1$ | $R^2$ | $R^3$ | $R^4$ | X | A | Z | Physikalische Daten |
|---|---|---|---|---|---|---|---|---|
| 9 | Cl | H | $CF_3$ | H | C-Cl | -CH-<br>$CH_3$ | COOH | |
| 10 | Cl | H | Cl | H | N | $-CH_2-$ | COOH | |
| 11 | Cl | H | Cl | H | N | -CH-<br>$CH_3$ | COOH | |
| 12 | Cl | H | $CF_3$ | H | C-Cl | $-CH_2-$ | COOH | |
| 13 | Cl | H | Cl | H | N | -CH-<br>$CH_3$ | $COOC_2H_5$ | |
| 14 | Cl | H | $CF_3$ | H | C-Cl | -CH-<br>$CH_3$ | COCl | |
| 15 | Cl | H | $CF_3$ | H | C-Cl | -CH-<br>$CH_3$ | $COO-CH_2-COOC_4H_9$ | |
| 16 | Cl | H | $CF_3$ | H | C-Cl | -CH-<br>$CH_3$ | $COOCHCOOC_2H_5$<br>$CH_3$ | |

Tabelle 1 - Fortsetzung

| Beisp.-Nr. | $R^1$ | $R^2$ | $R^3$ | $R^4$ | X | A | Z | Physikalische Daten |
|---|---|---|---|---|---|---|---|---|
| 17 | Cl | H | $CF_3$ | H | C-Cl | $-CH_2-$ | $COOCHCOOC_2H_5$<br>$CH_3$ | |
| 18 | Cl | Cl | $CF_3$ | H | C-Cl | $-CH_2-$ | $COSC_2H_5$ | |
| 19 | Cl | F | $CF_3$ | H | C-Cl | $-CH-$<br>$CH_3$ | $COSCH_2COOCH_3$ | |
| 20 | Cl | H | Cl | H | N | $-CH_2CH_2-$ | $CONHC_2H_5$ | |
| 21 | Cl | H | $CF_3$ | H | C-Cl | $-CH_2-$ | COCl | |
| 22 | Cl | H | $CF_3$ | H | C-Cl | $-CH_2CH_2-$ | COCl | |
| 23 | Cl | H | $CF_3$ | H | C-Cl | $-CH_2CH_2-$ | COOH | |
| 24 | Cl | H | Cl | H | N | $-CH_2CH_2-$ | COOH | |
| 25 | Cl | H | Cl | H | N | $-CH-$<br>$CH_3$ | $COOCH_2CH_2OCH_3$ | |
| 26 | Cl | H | $CF_3$ | H | C-Cl | $-CH-$<br>$CH_3$ | $COOCH_2CH_2SC_2H_5$ | |

Tabelle 1 - Fortsetzung

| Beisp.-Nr. | $R^1$ | $R^2$ | $R^3$ | $R^4$ | X | A | Z | Physikalische Daten |
|---|---|---|---|---|---|---|---|---|
| 27 | Cl | H | Cl | H | N | $-CH_2-$ | $COOCH_2-\overset{O}{\overset{\|}{P}}(OC_2H_5)_2$ | |
| 28 | Cl | H | $CF_3$ | H | C-Cl | $-CH_2-$ | $\underset{C_6H_5}{COOCH}-\overset{O}{\overset{\|}{P}}(OCH_3)_2$ | |
| 29 | Cl | H | $CF_3$ | H | C-Cl | $-CH_2CH_2-$ | $CONHCH(CH_3)_2$ | |
| 30 | Cl | Cl | $CF_3$ | H | C-Cl | $-CH_2-$ | $CON(C_2H_5)_2$ | |
| 31 | Cl | F | $CF_3$ | H | C-Cl | $-\underset{CH_3}{CH}-$ | $COOCH_2CH_2OCH_2-$ 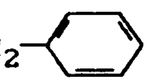 | |
| 32 | Cl | H | $CF_3$ | H | C-F | $-\underset{CH_3}{CH}-$ | $COOCH_2$ 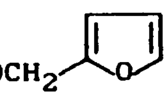 | |
| 33 | Cl | H | Cl | H | N | $-CH_2-$ | $CONHOCH_3$ | |

Tabelle 1 - Fortsetzung

| Beisp.-Nr. | $R^1$ | $R^2$ | $R^3$ | $R^4$ | X | A | Z | Physikalische Daten |
|---|---|---|---|---|---|---|---|---|
| 34 | Cl | H | $CF_3$ | H | C-Cl | $-CH_2CH_2-$ | $CO-NH-OC_2H_5$ | |
| 35 | Cl | Cl | $CF_3$ | H | C-Cl | -CH-<br>\|<br>$CH_3$ | $CONHCH_2COOC_2H_5$ | |
| 36 | CN | H | $CF_3$ | H | CH | $-CH_2-$ | $CONHNHSO_2CH_3$ | |
| 37 | Cl | H | Cl | H | N | $-CH_2-$ | $COOCH_2Si(CH_3)_3$ | |
| 38 | Cl | H | $CF_3$ | H | C-Cl | -CH-<br>\|<br>$CH_3$ | $COO(CH_2)_3OCH_2-$ 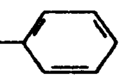 | |
| 39 | Cl | H | $CF_3$ | H | C-Cl | $-CH_2-$ | CN | |
| 40 | Cl | H | $CF_3$ | H | C-Cl | $-CH_2CH_2-$ | CN | |
| 41 | Cl | H | Cl | H | N | $-CH_2-$ | CN | |
| 42 | Cl | Cl | $CF_3$ | H | C-Cl | $-CH_2-$ | CN | |
| 43 | Cl | F | $CF_3$ | H | C-Cl | $-CH_2-$ | CN | |
| 44 | Cl | H | $CF_3$ | H | C-F | $-CH_2-$ | CN | |

<u>Tabelle 1</u> - Fortsetzung

| Beisp.-Nr. | $R^1$ | $R^2$ | $R^3$ | $R^4$ | X | A | Z | Physikalische Daten |
|---|---|---|---|---|---|---|---|---|
| 45 | Cl | Cl | $CF_3$ | H | C-Cl | $-\underset{\underset{CH_3}{\mid}}{CH}-$ | $COOC_2H_5$ | |
| 46 | Cl | H | $CF_3$ | H | C-Cl | $-\underset{\underset{CH_3}{\mid}}{CH}-$ | $COOCH_3$ | |
| 47 | Cl | H | $CF_3$ | H | C-Cl | $-CH_2CH_2-$ | $COOC_2H_5$ | |
| 48 | F | F | $CF_3$ | F | C-F | $-\underset{\underset{CH_3}{\mid}}{CH}-$ | $COOC_2H_5$ | |
| 49 | Cl | H | $CF_3$ | H | C-F | $-\underset{\underset{CH_3}{\mid}}{CH}-$ | COOH | |
| 50 | Cl | H | $CF_3$ | H | C-F | $-\underset{\underset{CH_3}{\mid}}{CH}-$ | $COOCH_2Si(CH_3)_3$ | |
| 51 | Cl | H | $CF_3$ | H | C-F | $-\underset{\underset{CH_3}{\mid}}{CH}-$ | $COOCH_2\overset{\overset{O}{\|}}{P}(OC_2H_5)_2$ | |

<u>Tabelle 1</u> - Fortsetzung

| Beisp.-Nr. | $R^1$ | $R^2$ | $R^3$ | $R^4$ | X | A | Z | Physikalische Daten |
|---|---|---|---|---|---|---|---|---|
| 52 | Cl | H | $CF_3$ | H | C-Cl | $-\underset{\displaystyle CH_3}{\underset{\vert}{CH}}-$ | $COOCH_2Si(CH_3)_3$ | |
| 53 | Cl | H | $CF_3$ | H | C-Cl | $-\underset{\displaystyle CH_3}{\underset{\vert}{CH}}-$ | $COOCH_2\overset{\displaystyle O}{\overset{\Vert}{P}}(OC_2H_5)_2$ | |
| 54 | Cl | H | $CF_3$ | H | C-Cl | $-\underset{\displaystyle CH_3}{\underset{\vert}{CH}}-$ | $COOCH_2CH_2OC_2H_5$ | |
| 55 | Cl | H | $CF_3$ | H | C-Cl | $-CH_2-$ | $CO-NH_2$ | |
| 56 | Cl | H | $CF_3$ | H | C-Cl | $-CH_2-$ | $CO-N(C_3H_7)_2$ | |
| 57 | Cl | H | $CF_3$ | H | C-Cl | $-CH_2-$ | $CO-N(CH_2CH=CH_2)_2$ | |
| 58 | Cl | H | $CF_3$ | H | C-Cl | $(-CH_2)_3-$ | $COOC_2H_5$ | |
| 59 | Cl | H | $CF_3$ | H | C-Cl | $-\underset{\displaystyle C_2H_5}{\underset{\vert}{CH}}-$ | $COOC_2H_5$ | |

Tabelle 1 - Fortsetzung

| Beisp.-Nr. | $R^1$ | $R^2$ | $R^3$ | $R^4$ | X | A | Z | Physikalische Daten |
|---|---|---|---|---|---|---|---|---|
| 60 | Cl | H | $CF_3$ | H | C-Cl | $-CH(CH_3)-$ | $COOCH_2-$ 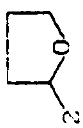 | |
| 61 | F | F | $CF_3$ | F | C-F | $-CH(CH_3)-$ | $COOCH_2-P(=O)(OCH_3)_2$ | |
| 62 | Cl | H | $CF_3$ | F | C-Cl | $-CH(CH_3)-$ | $COOCH(CH_3)-P(=O)(OCH_3)_2$ | |
| 63 | Cl | H | $CF_3$ | H | C-Cl | $-CH(C_2H_5)-$ | $COOC_2H_5$ | |
| 64 | Cl | H | $CF_3$ | H | N | $-CH(CH_3)-$ | COOH | |
| 65 | H | H | $CF_3$ | H | N | $-CH_2-$ | COOH | |
| 66 | Cl | H | $CF_3$ | H | CH | $-CH(CH_3)-$ | $COOCH_3$ | |

Ausgangsstoffe der Formel (II)

Beispiel (II-1)

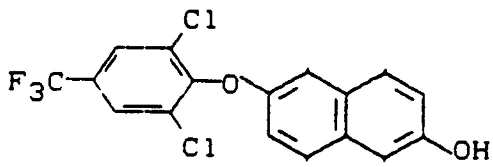

46,8 g (0,19 Mol) 3,4,5-Trichlor-benzotrifluorid werden zu einer auf 120 °C erwärmten Mischung aus 56,8 g (0,36 Mol) 2,6-Dihydroxy-naphthalin, 14,3 g (0,36 Mol) Natriumhydroxid-Pulver und 170 ml Dimethylsulfoxid langsam unter Rühren gegeben und das Reaktionsgemisch wird zunächst 3 Stunden bei 120 °C und dann noch ca. 15 Stunden bei 20 °C gerührt. Anschließend wird das Reaktionsgemisch auf das doppelte Volumen mit Wasser verdünnt und mit 1N-Salzsäure angesäuert. Nach Abtrennen des ausfallenden Feststoffes und Schütteln mit Toluol wird die organische Phase abgetrennt, mit Wasser gewaschen, mit Natriumsulfat getrocknet und filtriert. Vom Filtrat wird das Lösungsmittel im Wasserstrahlvakuum abdestilliert. Der Rückstand wird aus Ethanol umkristallisiert.

Man erhält 52 g (74 % der Theorie) 6-(2,6-Dichlor-4-trifluormethyl-phenoxy)-2-naphthol vom Schmelzpunkt 158 °C.

Analog Beispiel (II-1) können die in der nachstehenden Tabelle 2 aufgeführten Ausgangsstoffe der Formel (II) hergestellt werden.

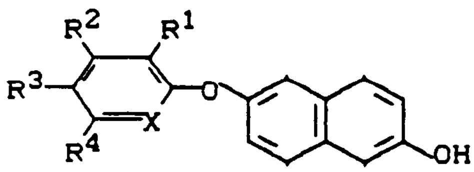

(II)

Tabelle 2: Beispiele für die Ausgangsstoffe der Formel (II)

| Beisp.-Nr. | $R^1$ | $R^2$ | $R^3$ | $R^4$ | X | Physikalische Daten |
|---|---|---|---|---|---|---|
| II-2 | Cl | H | $CF_3$ | H | C-H | Fp. : 107 °C |
| II-3 | H | H | $CF_3$ | H | C-H | |
| II-4 | Cl | Cl | $CF_3$ | H | C-Cl | |
| II-5 | Cl | F | $CF_3$ | H | C-Cl | |
| II-6 | Cl | H | $CF_3$ | H | C-F | |
| II-7 | CN | H | $CF_3$ | H | CH | |
| II-8 | Cl | H | Cl | H | N | |
| II-9 | Cl | H | $CF_3$ | H | N | |
| II-10 | H | H | $CF_3$ | H | N | |

Anwendungsbeispiele

In den folgenden Anwendungsbeispielen wird die nachstehend aufgeführte Verbindung als Vergleichssubstanz herangezogen:

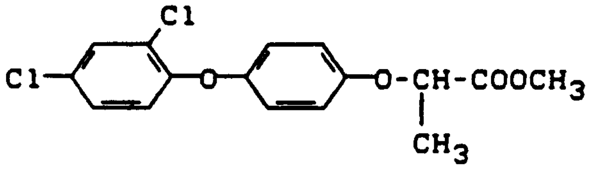

(A)

α-(4-(2,4-Dichlor-phenoxy)-phenoxy)-propionsäure-methylester
(bekannt aus DE-OS 22 23 894/Beispiel 86).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigen beispielsweise die Verbindungen gemäß Herstellungsbeispiel (1), (2) und (3) bei guter Selektivität in Mais und Weizen erheblich stärkere Wirkung gegen Problemunkräuter, wie z. B. Abutilon, Chenopodium, Galinsoga, Portulak und Viola, als die Vergleichssubstanz (A).

## Patentansprüche

1. (6-(Hetero)Aryloxy-naphthalin-2-yl-oxy)-alkancarbonsäure-Derivate der Formel (I)

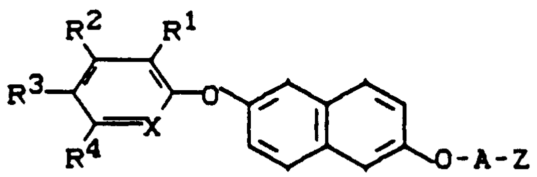

(I)

in welcher

- $R^1$ für Wasserstoff, Halogen, Cyano oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht,
- X für Stickstoff oder die Gruppierung C-$R^5$ steht, worin
  $R^5$ für Wasserstoff oder Halogen steht,
- A für gegebenenfalls verzweigtes Alkandiyl steht und
- Z für Cyano oder die Gruppierung -CO-Y steht, worin
  Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin
  $R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall- oder Erdalkalimetalläquivalent steht oder für die Gruppierung

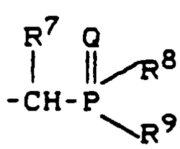

  steht, worin
  $R^7$ für Wasserstoff, Alkyl, Aryl, Furyl, Thienyl oder Pyridyl steht,
  $R^8$ für Alkyl oder Alkoxy steht,
  $R^9$ für Alkoxy steht und
  Q für Sauerstoff oder Schwefel steht,
  oder
  $R^6$ weiterhin für die Gruppierung $-(CH_2)_n-R^{10}$ steht, worin
  $R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl,

Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht,
sowie deren optisch aktive Isomere.

**2.** (6-(Hetero)Aryloxy-naphthalin-2-yl-oxy)-alkancarbonsäure-Derivate der Formel (I) gemäß Anspruch 1, in welcher

$R^1$ für Wasserstoff, Fluor, Chlor, Brom, Cyano oder Trifluormethyl steht,
$R^2$ für Wasserstoff, Fluor oder Chlor steht,
$R^3$ für Fluor, Chlor, Brom, Trifluormethyl, Trifluormethoxy, Trifluormethylthio oder Trifluormethylsulfonyl steht,
$R^4$ für Wasserstoff, Fluor oder Chlor steht,
X für Stickstoff oder die Gruppierung C-$R^5$ steht, worin
$R^5$ für Wasserstoff, Fluor, Chlor oder Brom steht,
A für gegebenenfalls verzweigtes $C_1$-$C_4$-Alkandiyl steht und
Z für Cyano oder die Gruppierung -CO-Y steht, worin
Y für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^6$ steht, worin
$R^6$ für einen gegebenenfalls durch Fluor und/ oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxycarbonyl-$C_1$-$C_2$-alkyl, $C_1$-$C_4$-Alkylamino-carbonyl-$C_1$-$C_2$-alkyl, Phenylaminocarbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium- oder Calcium-äquivalent steht, oder
für die Gruppierung

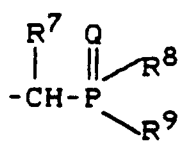

steht, worin
$R^7$ für Wasserstoff, $C_1$-$C_4$-Alkyl, Phenyl, Furyl, Thienyl oder Pyridyl steht,
$R^8$ für $C_1$-$C_4$-Alkyl oder $C_1$-$C_4$-Alkoxy steht,
$R^9$ für $C_1$-$C_4$-Alkoxy steht und
Q für Sauerstoff oder Schwefel steht,
oder
$R^6$ weiterhin für die Gruppierung $-(CH_2)_n-R^{10}$ steht, worin
n für die Zahlen 0, 1 oder 2 steht und
$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht,
sowie deren optisch aktive Isomere.

**3.** (6-(Hetero)Aryloxy-naphthalin-2-yl-oxy)-alkancarbonsäure-Derivate der Formel (I) gemäß Anspruch 1, in welcher

$R^1$ für Cyano, Fluor oder Chlor steht,
$R^2$ für Wasserstoff, Fluor oder Chlor steht,
$R^3$ für Chlor oder Trifluormethyl steht,

$R^4$ für Wasserstoff, Fluor oder Chlor steht,

X für Stickstoff oder die Gruppierung C-$R^5$ steht, worin

$R^5$ für Wasserstoff, Fluor oder Chlor steht,

A für Methylen ($-CH_2-$), Dimethylen ($-CH_2CH_2-$), Trimethylen ($(-CH_2-)_3$) oder Ethyliden

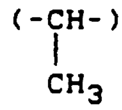

steht und

Z für Cyano oder die Gruppierung -CO-Y steht, worin

Y für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, Diallylamino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung -O-$R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl oder für ein Ammonium-, $C_1$-$C_3$-Alkylammonium-, Natrium-, oder Kalium-äquivalent steht, oder für die Gruppierung

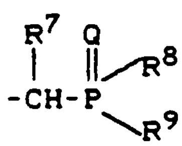

steht, worin

$R^7$ für Wasserstoff, Methyl, Phenyl, Furyl, Thienyl oder Pyridyl steht,

$R^8$ für Methoxy oder Ethoxy steht,

$R^9$ für Methoxy oder Ethoxy steht und

Q für Sauerstoff oder Schwefel steht

oder

$R^6$ weiterhin für die Gruppierung $(-CH_2-)_nR^{10}$ steht, worin

n für die Zahlen 0, 1 oder 2 steht und

$R^{10}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht.

**4.** Verfahren zur Herstellung von (6-(Hetero)Aryloxy-naphthalin-2-yl-oxy)-alkancarbonsäure-Derivaten der Formel (I),

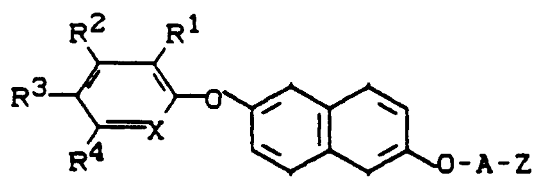

(I)

in welcher

$R^1$ für Wasserstoff, Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl

steht,

$R^4$ für Wasserstoff oder Halogen steht,

X für Stickstoff oder die Gruppierung C-$R^5$ steht, worin

$R^5$ für Wasserstoff oder Halogen steht,

A für gegebenenfalls verzweigtes Alkandiyl steht und

Z für Cyano oder die Gruppierung -CO-Y steht, worin

Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin

$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall- oder Erdalkalimetalläquivalent steht oder für die Gruppierung

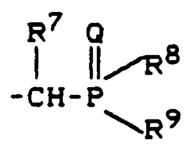

steht, worin

$R^7$ für Wasserstoff, Alkyl, Aryl, Furyl, Thienyl oder Pyridyl steht,

$R^8$ für Alkyl oder Alkoxy steht,

$R^9$ für Alkoxy steht und

Q für Sauerstoff oder Schwefel steht,

oder

$R^6$ weiterhin für die Gruppierung $-(CH_2)_n-R^{10}$ steht, worin

$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und

n für die Zahlen 0, 1 oder 2 steht,

dadurch gekennzeichnet, daß man

(a) 6-(Hetero)Aryloxy-2-naphthole der allgemeinen Formel (II)

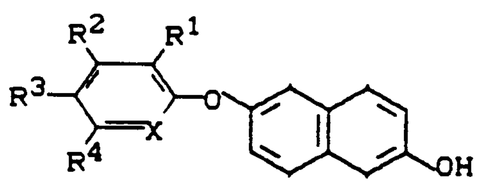

(II)

in welcher

$R^1$, $R^2$, $R^3$, $R^4$ und X die oben angegebenen Bedeutungen haben,

mit Carbonsäurederivaten der allgemeinen Formel (III)

$Z^1$ - A - Z (III)

in welcher

A und Z die oben angegebenen Bedeutungen haben und

$Z^1$ für eine nucleophile Abgangsgruppe steht,

gegebenenfalls in Gegenwart eines Säureakzeptors und gegebenenfalls in Gegenwart eines Verdünnungsmittels umsetzt, oder daß man

(b) Halogen-(hetero)aryl-Verbindungen der allgemeinen Formel (IV)

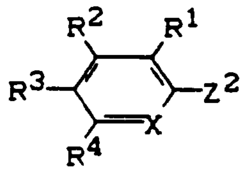

(IV)

in welcher

$R^1$, $R^2$, $R^3$, $R^4$ und X die oben angegebenen Bedeutungen haben und

$Z^2$ für Halogen steht,

mit (6-Hydroxy-naphthalin-2-yl-oxy)-alkancarbonsäure-Derivaten der allgemeinen Formel (V)

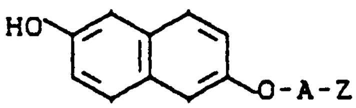

(V)

in welcher

A und Z die oben angegebenen Bedeutungen haben,

gegebenenfalls in Gegenwart eines Säureakzeptors und gegebenenfalls in Gegenwart eines Verdünnungsmittels umsetzt, oder daß man

(c) für den Fall, daß in der Formel (I) Z für die Gruppierung -CO-Y steht, worin Y für Hydroxy steht und A, $R^1$, $R^2$, $R^3$, $R^4$ und X die oben angegebenen Bedeutungen haben, Verbindungen der Formel (I), in welcher Z für Cyano oder für die Gruppierung -CO-Y steht, worin Y für Methoxy oder Ethoxy steht und A, $R^1$, $R^2$, $R^3$, $R^4$ und Y die oben angegebenen Bedeutungen haben,

mit einem Alkalihydroxid in Gegenwart eines organischen Lösungsmittels umsetzt und dann - gegebenenfalls nach Einengen - mit einer Mineralsäure ansäuert, oder daß man

(d) für den Fall, daß in der Formel (I) Z für die Gruppierung -CO-Y steht, worin Y für Halogen steht und A, $R^1$, $R^2$, $R^3$, $R^4$ und X die oben angegebenen Bedeutungen haben, Verbindungen der allgemeinen Formel (I), in welcher Z für die Gruppierung -CO-Y steht, worin Y für Hydroxy steht und A, $R^1$, $R^2$, $R^3$, $R^4$ und X die oben angegebenen Bedeutungen haben, mit einem Halogenierungsmittel gegebenenfalls in Gegenwart eines Verdünnungsmittels und gegebenenfalls in Gegenwart eines Katalysators umsetzt, oder daß man

(e) für den Fall, daß in der Formel (I) Z für die Gruppierung -CO-Y steht, worin Y mit Ausnahme von Halogen die oben angegebene Bedeutung hat und A, $R^1$, $R^2$, $R^3$, $R^4$ und X die oben angegebenen Bedeutungen haben, Verbindungen der allgemeinen Formel (I), in welcher Z für die Gruppierung -CO-Y steht, worin Y für Halogen steht und A, $R^1$, $R^2$, $R^3$, $R^4$ und X die oben angegebenen Bedeutungen haben,

mit Verbindungen der allgemeinen Formel (VI)

H - Y (VI)

in welcher

Y mit Ausnahme von Halogen die oben angegebene Bedeutung hat,

gegebenenfalls in Gegenwart eines Säureakzeptors und gegebenenfalls in Gegenwart eines Verdünnungsmittels umsetzt, oder daß man

(f) für den Fall, daß in der Formel (I) Z für die Gruppierung -CO-Y steht, worin Y für die Gruppierung $-O-R^6$ steht, worin $R^6$ mit Ausnahme von Ammonium, Alkylammonium, Alkalimetall und Erdalkalimetall die oben angegebene Bedeutung hat und A, $R^1$, $R^2$, $R^3$, $R^4$ und X die oben angegebenen Bedeutungen haben, Verbindungen der allgemeinen Formel (I), in welcher Z für die Gruppierung -CO-Y steht, worin Y für Hydroxy steht und A, $R^1$, $R^2$, $R^3$, $R^4$ und Y die oben angegebenen

Bedeutungen haben,
mit Verbindungen der allgemeinen Formel (VII)

$Z^3$ - $R^{6-1}$ (VII)

in welcher

$R^{6-1}$ mit Ausnahme von Ammonium, Alkylammonium, Alkalimetall und Erdalkalimetall die oben für $R^6$ angegebene Bedeutung hat und

$Z^3$ für Halogen steht,

gegebenenfalls in Gegenwart eines Säureakzeptors und gegebenenfalls in Gegenwart eines Verdünnungsmittels umsetzt, oder daß man

(g) für den Fall, daß in der Formel (I) Z für die Gruppierung -CO-Y steht, worin Y für die Gruppierung -O-$R^6$ steht, worin $R^6$ mit Ausnahme von Ammonium, Alkylammonium, Alkalimetall und Erdalkalimetall die oben angegebene Bedeutung hat, und A, $R^1$, $R^2$, $R^3$, $R^4$ und X die oben angegebenen Bedeutungen haben, Verbindungen der Formel (I), in welcher Z für Cyano steht und A, $R^1$, $R^2$, $R^3$, $R^4$ die oben angegebenen Bedeutungen haben
mit Hydroxylverbindungen der allgemeinen Formel (VIII)

HO-$R^{6-2}$ (VIII)

in welcher

$R^{6-2}$ für $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Phenylthio-$C_1$-$C_3$-alkyl, Benzyloxy-$C_1$-$C_3$-alkyl, Benzylthio-$C_1$-$C_3$-alkyl und $C_1$-$C_4$-Alkoxycarbonyl-$C_1$-$C_2$-alkyl steht,

in Gegenwart einer Mineralsäure und gegebenenfalls in Gegenwart eines Verdünnungsmittels umsetzt.

**5.** Herbizide Mittel, gekennzeichnet durch einen Gehalt an mindestens einem (6-(Hetero)Aryloxy-naphthalin-2-yl-oxy)-alkancarbonsäure-Derivat der Formel (I) gemäß den Ansprüchen 1 bis 4.

**6.** Verfahren zur Bekämpfung von Unkräutern, dadurch gekennzeichnet, daß man (6-(Hetero)Aryloxy-naphthalin-2-yl-oxy)-alkancarbonsäure-Derivate der Formel (I) gemäß den Ansprüchen 1 bis 4 auf die Unkräuter und/oder ihren Lebensraum einwirken läßt.

**7.** Verwendung von (6-(Hetero)Aryloxy-naphthalin-2-yl-oxy)-alkancarbonsäure-Derivaten der Formel (I) gemäß den Ansprüchen 1 bis 4 zur Bekämpfung von Unkräutern.

**8.** Verfahren zur Herstellung von herbiziden Mitteln, dadurch gekennzeichnet, daß man (6-(Hetero)Aryloxy-naphthalin-2-yl-oxy)-alkancarbonsäure-Derivate der Formel (I) gemäß den Ansprüchen 1 bis 4 mit Streckmitteln und/oder oberflächenaktiven Substanzen vermischt.

**9.** 6-(Hetero)Aryloxy-2-naphthole der Formel (II)

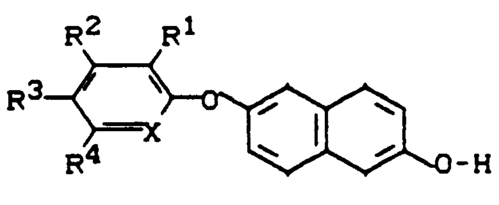

(II)

in welcher

$R^1$ für Wasserstoff, Halogen, Cyano oder Trifluormethyl steht,

$R^2$ für Wasserstoff oder Halogen steht,

$R^3$ für Halogen, Trifluormethyl, Trifluormethoxy, Trifluormethylthio oder Trifluormethylsulfonyl steht,

$R^4$ für Wasserstoff oder Halogen steht und

X für Stickstoff oder die Gruppierung C-$R^5$ steht, worin

$R^5$ für Wasserstoff oder Halogen steht.

**10.** Verfahjren zur Herstellung von 6-(Hetero)Aryloxy-2-naphtholen der Formel (II)

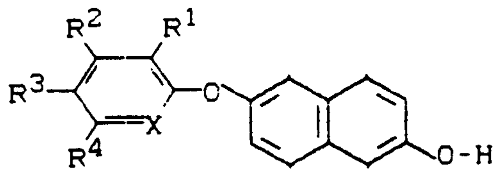

(II)

in welcher

- $R^1$ für Wasserstoff, Halogen, Cyano oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht und
- X für Stickstoff oder die Gruppierung C-$R^5$ steht, worin
  $R^5$ für Wasserstoff oder Halogen steht,

dadurch gekennzeichnet, daß man Halogen(hetero)aryl-Verbindungen der Formel (IV)

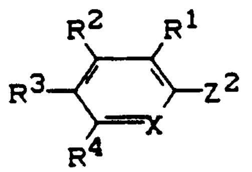

(IV)

in welcher

- $R^1$, $R^2$, $R^3$, $R^4$ und X die oben angegebenen Bedeutungen haben und
- $Z^2$ für Halogen steht,

mit 2,6-Dihydroxynaphthalin in Gegenwart eines Säureakzeptors und in Gegenwart eines Verdünnungsmittels umsetzt.

## Claims

**1.** (6-(Hetero)aryloxy-naphthalen-2-yl-oxy)-alkane-carboxylic acid derivatives of the formula (I)

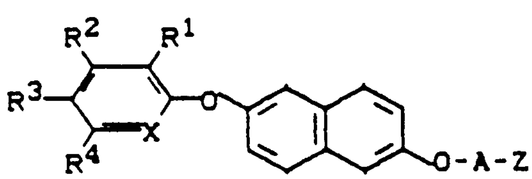

(I)

in which

- $R^1$ represents hydrogen, halogen, cyano or trifluoromethyl,
- $R^2$ represents hydrogen or halogen,
- $R^3$ represents halogen, trifluoromethyl, trifluoromethoxy, trifluoromethylthio or trifluoromethylsulphonyl,
- $R^4$ represents hydrogen or halogen,
- X represents nitrogen or the grouping C-$R^5$, wherein
  $R^5$ represents hydrogen or halogen,
- A represents optionally branched alkanediyl and
- Z represents cyano or the grouping -CO-Y, wherein

Y represents halogen, hydroxyl, amino, alkylamino, alkenylamino, alkinylamino, arylamino, aralkylamino, alkoxycarbonylalkylamino, cyanamino, dialkylamino, dialkenylamino, alkylsulphonylamino, arylsulphonylamino, hydroxyamino, alkoxyamino, hydrazino, alkylsulphonylhydrazino, arylsulphonylhydrazino, alkylthio, arylthio, aralkylthio, alkoxycarbonylalkylthio or the grouping -O-$R^6$, wherein

$R^6$ represents an optionally halogen-substituted radical from the series alkyl, alkenyl, alkinyl, alkoxyalkyl, alkylthioalkyl, alkylsulphinylalkyl, alkylsulphonylalkyl, aryloxyalkyl, arylthioalkyl, arylalkoxyalkyl, arylalkylthioalkyl, trialkylsilylalkyl, alkoxycarbonylalkyl, alkylaminocarbonylalkyl, arylaminocarbonylalkyl, N-alkyl-N-aryl-aminocarbonylalkyl, aralkyl, azolylalkyl and alkylideneamino, or an ammonium, alkylammonium, alkali metal or alkaline earth metal equivalent, or the grouping

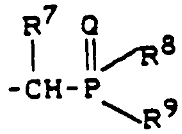

wherein

$R^7$ represents hydrogen, alkyl, aryl, furyl, thienyl or pyridyl,

$R^8$ represents alkyl or alkoxy,

$R^9$ represents alkoxy and

Q represents oxygen or sulphur,

or

$R^6$ furthermore represents the grouping $-(CH_2)_n-R^{10}$, wherein

$R^{10}$ represents an optionally halogen-and/or alkyl-substituted heterocyclic radical from the series furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl, and

n represents the numbers 0, 1 or 2,

and optically active isomers thereof.

**2.** (6-(Hetero)aryloxy-naphthalen-2-yl-oxy)-alkane-carboxylic acid derivatives of the formula (I) according to Claim 1, in which

$R^1$ represents hydrogen, fluorine, chlorine, bromine, cyano or trifluoromethyl,

$R^2$ represents hydrogen, fluorine or chlorine,

$R^3$ represents fluorine, chlorine, bromine, trifluoromethyl, trifluoromethoxy, trifluoromethylthio or trifluoromethylsulphonyl,

$R^4$ represents hydrogen, fluorine or chlorine,

X represents nitrogen or the grouping C-$R^5$, wherein

$R^5$ represents hydrogen, fluorine, chlorine or bromine,

A represents optionally branched $C_1$-$C_4$-alkanediyl and

Z represents cyano or the grouping -CO-Y, wherein

Y represents chlorine, hydroxyl, amino, $C_1$-$C_6$-alkylamino, $C_3$-$C_4$-alkenylamino, $C_3$-$C_4$-alkinylamino, phenylamino, benzylamino, $C_1$-$C_4$-alkoxycarbonyl-$C_1$-$C_2$-alkylamino, cyanamino, di-($C_1$-$C_4$-alkyl)-amino, di-($C_3$-$C_4$-alkenyl)-amino, $C_1$-$C_4$-alkylsulphonylamino, phenylsulphonylamino, tolylsulphonylamino, hydroxyamino, $C_1$-$C_6$-alkoxyamino, hydrazino, $C_1$-$C_4$-alkylsulphonylhydrazino, phenylsulphonylhydrazino, tolylsulphonylhydrazino, $C_1$-$C_4$-alkylthio, phenylthio, benzylthio, $C_1$-$C_4$-alkoxy-carbonyl-$C_1$-$C_2$-alkylthio or the grouping -O-$R^5$, wherein

$R^6$ represents an optionally fluorine-and/or chlorine-substituted radical from the series $C_1$-$C_6$-alkyl, $C_3$-$C_4$-alkenyl, $C_3$-$C_4$-alkinyl, $C_1$-$C_4$-alkoxy-$C_1$-$C_4$-alkyl, $C_1$-$C_4$-alkylthio-$C_1$-$C_4$-alkyl, $C_1$-$C_4$-alkylsulphinyl-$C_1$-$C_4$-alkyl, $C_1$-$C_4$-alkylsulphonyl-$C_1$-$C_4$-alkyl, phenoxy-$C_1$-$C_3$-alkyl, trimethylsilylmethyl, phenylthio-$C_1$-$C_3$-alkyl, benzyloxy-$C_1$-$C_3$-alkyl, benzylthio-$C_1$-$C_3$-alkyl, $C_1$-$C_4$-alkoxy-carbonyl-$C_1$-$C_2$-alkyl, $C_1$-$C_4$-alkylamino-carbonyl-$C_1$-$C_2$-alkyl, phenylaminocarbonyl-$C_1$-$C_4$-alkyl, N-($C_1$-$C_4$-alkyl)-N-phenylaminocarbonyl-$C_1$-$C_4$-alkyl, benzyl, pyrazolyl-$C_1$-$C_4$-alkyl and $C_2$-$C_4$-alkylideneamino, or an ammonium, a $C_1$-$C_4$-alkylam-

monium, a sodium, potassium or calcium equivalent, or the grouping

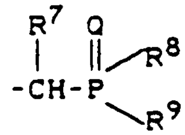

wherein
$R^7$ represents hydrogen, $C_1$-$C_4$-alkyl, phenyl, furyl, thienyl or pyridyl,
$R^8$ represents $C_1$-$C_4$-alkyl or $C_1$-$C_4$-alkoxy,
$R^9$ represents $C_1$-$C_4$-alkoxy and
Q represents oxygen or sulphur,
or
$R^6$ furthermore represents the grouping $-(CH_2)_nR^{10}$, wherein
n represents the numbers 0, 1 or 2 and
$R^{10}$ represents an optionally fluorine-, chlorine-, bromine- and/or $C_1$-$C_4$-alkyl-substituted heterocyclic radical from the series furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl,
and optically active isomers thereof.

**3.** (6-(Hetero)aryloxy-naphthalen-2-yl-oxy)-alkanecarboxylic acid derivatives of the formula (I) according to Claim 1, in which
$R^1$ represents cyano, fluorine or chlorine,
$R^2$ represents hydrogen, fluorine or chlorine,
$R^3$ represents chlorine or trifluoromethyl,
$R^4$ represents hydrogen, fluorine or chlorine,
X represents nitrogen or the grouping C-$R^5$, wherein
$R^5$ represents hydrogen, fluorine or chlorine,
A represents methylene ($-CH_2-$), dimethylene ($-CH_2CH_2-$), trimethylene ($(-CH_2-)_3$) or ethylidene

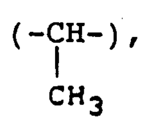

and
Z represents cyano or the grouping -CO-Y, wherein
Y represents chlorine, hydroxyl, amino, $C_1$-$C_4$-alkylamino, phenylamino, $C_1$-$C_4$-alkoxycarbonyl-$C_1$-$C_2$-alkylamino, di-($C_1$-$C_3$-alkyl)-amino, diallylamino, $C_1$-$C_4$-alkylsulphonylamino, phenylsulphonylamino, hydroxyamino, cyanamino, $C_1$-$C_4$-alkoxyamino, hydrazino, $C_1$-$C_4$-alkylsulphonylhydrazino, phenylsulphonylhydrazino, $C_1$-$C_4$-alkylthio or $C_1$-$C_4$-alkoxy-carbonyl-$C_1$-$C_2$-alkylthio, or the grouping -O-$R^6$, wherein
$R^6$ represents $C_1$-$C_4$-alkyl, $C_1$-$C_2$-alkoxy-$C_1$-$C_2$-alkyl, $C_1$-$C_2$-alkylthio-$C_1$-$C_2$-alkyl, $C_1$-$C_2$-alkylsulphinyl-$C_1$-$C_2$-alkyl, $C_1$-$C_2$-alkylsulphonyl-$C_1$-$C_2$-alkyl, benzyloxy-$C_1$-$C_3$-alkyl, benzylthio-$C_1$-$C_3$-alkyl, $C_1$-$C_4$-alkoxycarbonyl-$C_1$-$C_2$-alkyl, $C_1$-$C_4$-alkylaminocarbonyl-$C_1$-$C_2$-alkyl, benzyl and trimethylsilylmethyl, or an ammonium, $C_1$-$C_3$-alkylammonium, sodium or potassium equivalent, or the grouping

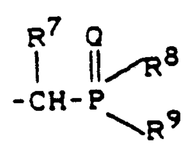

wherein
$R^7$ represents hydrogen, methyl, phenyl, furyl, thienyl or pyridyl,
$R^8$ represents methoxy or ethoxy,
$R^9$ represents methoxy or ethoxy and
Q represents oxygen or sulphur,
or
$R^6$ furthermore represents the grouping $(-CH_2-)_nR^{10}$, wherein
n represents the numbers 0, 1 or 2 and
$R^{10}$ represents an optionally chlorine- and/or methyl-substituted heterocyclic radical from the series furyl, tetrahydrofuryl, thienyl, perhydropyranyl, oxazolyl, thiazolyl and dioxolanyl.

**4.** Process for the preparation of (6-(hetero)aryloxynaphthalen-2-yl-oxy)-alkanecarboxylic acid derivatives of the formula (I),

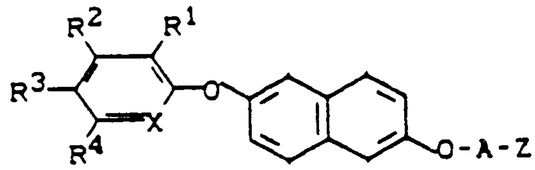

(I)

in which
$R^1$ represents hydrogen, halogen, cyano or trifluoromethyl,
$R^2$ represents hydrogen or halogen,
$R^3$ represents halogen, trifluoromethyl, trifluoromethoxy, trifluoromethylthio or trifluoromethylsulphonyl,
$R^4$ represents hydrogen or halogen,
X represents nitrogen or the grouping $C-R^5$, wherein
$R^5$ represents hydrogen or halogen,
A represents optionally branched alkanediyl and
Z represents cyano or the grouping -CO-Y, wherein
Y represents halogen, hydroxyl, amino, alkylamino, alkenylamino, alkinylamino, arylamino, aralkylamino, alkoxycarbonylalkylamino, cyanamino, dialkylamino, dialkenylamino, alkylsulphonylamino, arylsulphonylamino, hydroxyamino, alkoxyamino, hydrazino, alkylsulphonylhydrazino, arylsulphonylhydrazino, alkylthio, arylthio, aralkylthio, alkoxycarbonylalkylthio or the grouping $-O-R^6$, wherein
$R^6$ represents an optionally halogen-substituted radical from the series alkyl, alkenyl, alkinyl, alkoxyalkyl, alkylthioalkyl, alkylsulphinylalkyl, alkylsulphonylalkyl, aryloxyalkyl, arylthioalkyl, arylalkoxyalkyl, arylalkylthioalkyl, trialkylsilylalkyl, alkoxycarbonylalkyl, alkylaminocarbonylalkyl, arylaminocarbonylalkyl, N-alkyl-N-aryl-aminocarbonylalkyl, aralkyl, azolylalkyl and alkylideneamino, or an ammonium, alkylammonium, alkali metal or alkaline earth metal equivalent, or the grouping

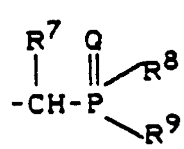

wherein
$R^7$ represents hydrogen, alkyl, aryl, furyl, thienyl or pyridyl,
$R^8$ represents alkyl or alkoxy,
$R^9$ represents alkoxy and
Q represents oxygen or sulphur,
or
$R^6$ furthermore represents the grouping $-(CH_2)_n-R^{10}$, wherein

$R^{10}$ represents an optionally halogen- and/or alkyl-substituted heterocyclic radical from the series furyl, tetrahydrofuryl, oxotetrahydrofuryl, thienyl, tetrahydrothienyl, perhydropyranyl, oxazolyl, thiazolyl, thiadiazolyl, dioxolanyl, perhydropyrrolyl, oxoperhydropyrrolyl, pyridinyl or pyrimidinyl, and

n represents the numbers 0, 1 or 2,

characterised in that

a) 6-(hetero)aryloxy-2-naphthols of the general formula (II)

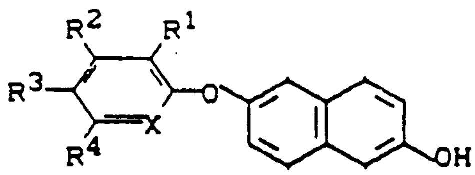

(II)

in which

$R^1$, $R^2$, $R^3$, $R^4$ and X have the abovementioned meanings,

are reacted with carboxylic acid derivatives of the general formula (III)

$Z^1$ - A - Z (III)

in which

A and Z have the abovementioned meanings and

$Z^1$ represents a nucleophilic leaving group,

if appropriate in the presence of an acid acceptor and if appropriate in the presence of a diluent, or

(b) halogeno-(hetero)aryl compounds of the general formula (IV)

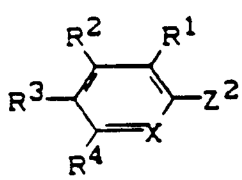

(IV)

in which,

$R^1$, $R^2$, $R^3$, $R^4$ and X have the abovementioned meanings and

$Z^2$ represents halogen,

are reacted with (6-hydroxy-naphthalen-2-yl-oxy)-alkane-carboxylic acid derivatives of the general formula (V)

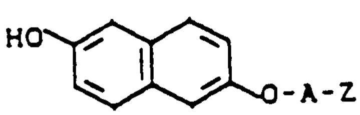

(V)

in which

A and Z have the abovementioned meanings,

if appropriate in the presence of an acid acceptor and if appropriate in the presence of a diluent, or

(c) in the event that, in the formula (I), Z represents the grouping -CO-Y, wherein Y represents hydroxyl and A, $R^1$, $R^2$, $R^3$, $R^4$ and X have the abovementioned meanings, compounds of the formula (I) in which Z represents cyano or the grouping -CO-Y, wherein Y represents methoxy or ethoxy and A, $R^1$, $R^2$, $R^3$, $R^4$ and Y have the abovementioned meanings,

are reacted with an alkali metal hydroxide in the presence of an organic solvent, the reaction mixture

is concentrated, if appropriate, and then acidified with a mineral acid, or
(d) in the event that, in the formula (I), Z represents the grouping -CO-Y, wherein Y represents halogen and A, $R^1$, $R^2$, $R^3$, $R^4$ and X have the abovementioned meanings, compounds of the general formula (I) in which Z represents the grouping -CO-Y, wherein Y represents hydroxyl and A, $R^1$, $R^2$, $R^3$, $R^4$ and X have the abovementioned meanings, are reacted with a halogenating agent, if appropriate in the presence of a diluent and if appropriate in the presence of a catalyst, or
(e) in the event that, in the formula (I), Z represents the grouping -CO-Y, wherein Y has the meaning given above with the exception of halogen and A, $R^1$, $R^2$, $R^3$, $R^4$ and X have the abovementioned meanings, compounds of the general formula (I) in which Z represents the grouping -CO-Y, wherein Y represents halogen and A, $R^1$, $R^2$, $R^3$, $R^4$ and X have the abovementioned meanings,
are reacted with compounds of the general formula (VI)

H - Y (VI)

in which
Y has the meaning given above with the exception of halogen,
if appropriate in the presence of an acid acceptor and if appropriate in the presence of a diluent, or
(f) in the event that, in the formula (I), Z represents the grouping -CO-Y, wherein Y represents the grouping -O-$R^6$, wherein $R^6$ has the abovementioned meaning with the exception of ammonium, alkylammonium, alkali metal and alkaline earth metal, and A, $R^1$, $R^2$, $R^3$, $R^4$ and X have the abovementioned meanings, compounds of the general formula (I) in which Z represents the grouping -CO-Y, wherein Y represents hydroxyl and A, $R^1$, $R^2$, $R^3$, $R^4$ and Y have the abovementioned meanings,
are reacted with compounds of the general formula (VII)

$Z^3$ - $R^{6-1}$ (VII)

in which
$R^{6-1}$ has the meaning given above for $R^6$, with the exception of ammonium, alkylammonium, alkali metal and alkaline earth metal, and
$Z^3$ represents halogen,
if appropriate in the presence of an acid acceptor and if appropriate in the presence of a diluent, or
(g) in the event that, in the formula (I), Z represents the grouping -CO-Y, wherein Y represents the grouping -O-$R^6$, wherein $R^6$ has the abovementioned meaning, with the exception of ammonium, alkylammonium, alkali metal and alkaline earth metal, and A, $R^1$, $R^2$, $R^3$, $R^4$ and X have the abovementioned meanings, compounds of the formula (I) in which Z represents cyano and A, $R^1$, $R^2$, $R^3$ and $R^4$ have the abovementioned meanings
are reacted with hydroxyl compounds of the general formula (VIII)

HO-$R^{6-2}$ (VIII)

in which
$R^{6-2}$ represents $C_1$-$C_6$-alkyl, $C_3$-$C_4$-alkenyl, $C_3$-$C_4$-alkinyl, $C_1$-$C_4$-alkoxy-$C_1$-$C_4$-alkyl, $C_1$-$C_4$-alkylthio-$C_1$-$C_4$-alkyl, phenoxy-$C_1$-$C_3$-alkyl, phenylthio-$C_1$-$C_3$-alkyl, benzyloxy-$C_1$-$C_3$-alkyl, benzylthio-$C_1$-$C_3$-alkyl and $C_1$-$C_4$-alkoxycarbonyl-$C_1$-$C_2$-alkyl,
in the presence of a mineral acid and if appropriate in the presence of a diluent.

**5.** Herbicidal agents, characterised in that they contain at least one (6-(hetero)aryloxy-naphthalen-2-yl-oxy)-alkanecarboxylic acid derivative of the formula (I) according to Claims 1 to 4.

**6.** Method for combating weeds, characterised in that (6-(hetero)aryloxy-naphthalen-2-yl-oxy)-alkanecarboxylic acid derivatives of the formula (I) according to Claims 1 to 4 are allowed to act on the weeds and/or their environment.

**7.** Use of (6(hetero)aryloxy-naphthalen-2-yl-oxy)-alkanecarboxylic acid derivatives of the formula (I) according to Claims 1 to 4 for combating weeds.

**8.** Process for the preparation of herbicidal agents, characterised in that (6-(hetero)aryloxy-naphthalen-2-yl-oxy)-alkanecarboxylic acid derivatives of the formula (I) according to Claims 1 to 4 are mixed with extenders and/or surfactants.

**9.** 6-(Hetero)aryloxy-2-naphthols of the formula (II)

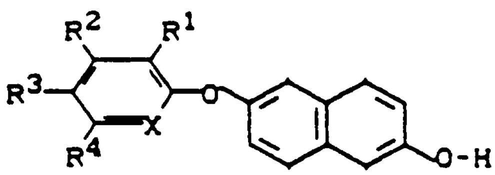

(II)

in which

$R^1$ represents hydrogen, halogen, cyano or trifluoromethyl,

$R^2$ represents hydrogen or halogen,

$R^3$ represents halogen, trifluoromethyl, trifluoromethoxy, trifluoromethylthio or trifluoromethylsulphonyl,

$R^4$ represents hydrogen or halogen and

X represents nitrogen or the grouping C-$R^5$, wherein

$R^5$ represents hydrogen or halogen.

**10.** Process for the preparation of 6-(hetero)aryloxy-2-naphthols of the formula (II)

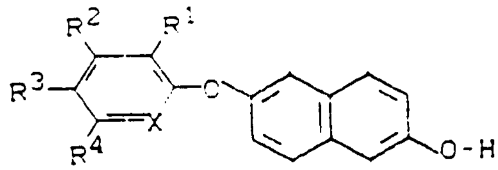

(II)

in which

$R_1$ represents hydrogen, halogen, cyano or trifluoromethyl,

$R^2$ represents hydrogen or halogen,

$R^3$ represents halogen, trifluoromethyl, trifluoromethoxy, trifluoromethylthio or trifluoromethylsulphonyl,

$R^4$ represents hydrogen or halogen and

X represents nitrogen or the grouping C-$R^5$, wherein

$R^5$ represents hydrogen or halogen,

characterized in that halogeno-(hetero)-aryl compounds of the formula (IV)

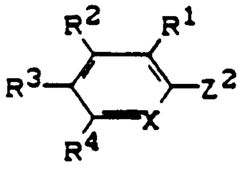

(IV)

in which

$R^1$, $R^2$, $R^3$, $R^4$ and X have the abovementioned meanings and

$Z^2$ represents halogen,

are reacted with 2,6-dihydroxynaphthalene in the presence of an acid acceptor and in the presence of a diluent.

**Revendications**

1. Dérivés d'acides (6-(hétéro)aryloxy-naphtalène-2-yl-oxy)-alcane-carboxyliques de formule I

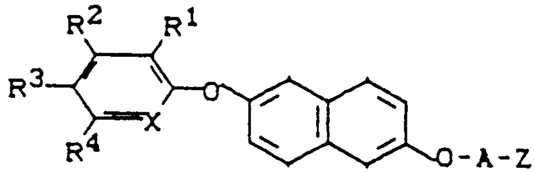

(I)

dans laquelle
$R^1$ représente l'hydrogène, un halogène, un groupe cyano ou trifluorométhyle,
$R^2$ représente l'hydrogène ou un halogène,
$R^3$ représente un halogène, un groupe trifluorométhyle, trifluorométhoxy, trifluorométhylthio ou trifluorométhylsulfonyle,
$R^4$ représente l'hydrogène ou un halogène,
X représente l'azote ou le groupe C-$R^5$ dans lequel $R^5$ représente l'hydrogène ou un halogène,
A représente un groupe alcane-diyle éventuellement ramifié, et
Z représente un groupe cyano ou le groupement -CO-Y dans lequel
Y représente un halogène, un groupe hydroxy, amino, alkylamino, alcénylamino, alcynylamino, arylamino, aralkylamino, alcoxycarbonylalkylamino, cyanamino, dialkylamino, dialcénylamino, alkylsulfonylamino, arylsulfonylamino, hydroxyamino, alcoxyamino, hydrazino, alkylsulfonylhydrazino, arylsulfonylhydrazino, alkylthio, arylthio, aralkylthio, alcoxycarbonylalkylthio ou le groupement -O-$R^6$ dans lequel
$R^6$ représente un radical, éventuellement substitué par des halogènes, choisi parmi les radicaux alkyle, alcényle, alcynyle, alcoxyalkyle, alkythio-alkyle, alkylsulfinylalkyle, alkylsufonylalkyle, aryloxyalkyle, arylthio-alkyle, arylalcoxyalkyle, arylalkylthioalkyle, trialkylsilylalkyle, alcoxycarbonylalkyle, alkylaminocarbonylalkyle, arylaminocarbonylalkyle, N-alkyl-N-arylaminocarbonylalkyle, aralkyl, azolylalkyle, alkylidène-amino ou un équivalent d'ammonium, d'alkylammonium, de métal alcalin ou alcalino-terreux, ou bien le groupement

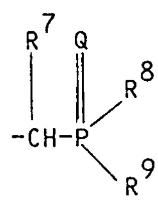

dans lequel
$R^7$ représente l'hydrogène, un groupe alkyle, aryle, furyle, thiényle ou pyridyle,
$R^8$ représente un groupe alkyle ou alcoxy,
$R^9$ représente un groupe alcoxy, et
Q représente l'oxygène ou le soufre, ou bien
$R^6$ représente le groupement $-(CH_2)_n-R^{10}$ dans lequel
$R^{10}$ représente un radical hétérocyclique éventuellement substitué par des halogènes et/ou des groupes alkyle et choisi parmiles radicaux furyle, tétrahydrofuryle, oxotétrahydrofuryle, thiényle, tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle, et
n est égal à 0, 1 ou 2,
et leurs isomères optiques.

2. Dérivés d'acides (6-(hétéro)aryloxy-naphtalène-2-yl-oxy)-alcane-carboxyliques de formule I de la revendication 1 dans laquelle
$R^1$ représente l'hydrogène, le fluor, le chlore, le brome, un groupe cyano ou trifluorométhyle,
$R^2$ représente l'hydrogène, le fluor ou le chlore,
$R^3$ représente le fluor, le chlore, le brome, un groupe trifluorométhyle, trifluorométhoxy, trifluorométhyl-

thio ou trifluorométhylsulfonyle,
$R^4$ représente l'hydrogène, le fluor ou le chlore,
X représente l'azote ou le groupement C-$R^5$
$R^5$ représente l'hydrogène, le fluor, le chlore ou le brome,
A représente un groupe alcane-diyle en $C_1$-$C_4$ éventuellement ramifié,
Z représente un groupe cyano ou le groupement -CO-Y dans lequel
Y représente le chlore, un groupe hydroxy, amino, alkylamino en $C_1$-$C_6$, alcényl amino en $C_3$-$C_4$, alcynylamino en $C_3$-$C_4$, phénylamino, benzylamino, (alcoxy en $C_1$-$C_4$)-carbonyl-alkylamino en $C_1$-$C_2$, cyanamino, di-(alkyle en $C_1$-$C_4$)-amino, di-(alcényle en $C_3$-$C_4$)-amino, alkylsulfonylamino en $C_1$-$C_4$, phénylsulfonylamino, tolylsulfonylamino, hydroxyamino, alcoxyamino en $C_1$-$C_6$, hydrazino, (alkyle en $C_1$-$C_4$)-sulfonylhydrazino, phénylsulfonylhydrazino, tolylsulfonylhydrazino, alkylthio en $C_1$-$C_4$, phénylthio, benzylthio, (alcoxy en $C_1$-$C_4$)-carbonyl-alkylthio en $C_1$-$C_2$ ou le groupement -O-$R^6$ dans lequel
$R^6$ représente un radical éventuellement substitué par le fluor et/ou le chlore et choisi parmi les radicaux alkyle en $C_1$-$C_6$,alcényle en $C_3$-$C_4$, alcynyle en $C_3$-$C_4$, (alcoxy en $C_1$-$C_4$)-alkyle en $C_1$-$C_4$, (alkylthio en $C_1$-$C_4$)-alkyle en $C_1$-$C_4$, (alkyle en $C_1$-$C_4$)-sulfinylalkyle en $C_1$-$C_4$, (alkyle en ($C_1$-$C_4$)-sulfonylalkyle en $C_1$-$C_4$, phénoxy-alkyle en alkyle en $C_1$-$C_3$, triméthylsilylméthyle, phénylthio-alkyle en $C_1$-$C_3$, benzyloxy-alkyle en $C_1$-$C_3$, benzylthio-alkyle en $C_1$-$C_3$, (alcoxy en $C_1$-$C_4$)-carbonylalkyle en $C_1$-$C_2$, (alkylamino en $C_1$-$C_4$)-carbonylalkyle en $C_1$-$C_2$, phénylaminocarbonylalkyle en $C_1$-$C_4$, N-(alkyle en $C_1$-$C_4$)-N-phénylaminocarbonylalkyle en $C_1$-$C_4$, benzyle, pyrazolyl-alkyle en $C_1$-$C_4$, alkylidène-amino en $C_2$-$C_4$ ou un équivalent d'ammonium, d'alkylammonium en $C_1$-$C_4$, de sodium, de potassium ou de calcium, ou bien le groupement

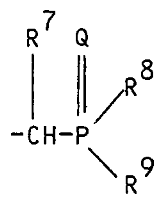

dans lequel
$R^7$ représente l'hydrogène, un groupe alkyle en $C_1$-$C_4$, phényle, furyle, thiényle ou pyridyle,
$R^8$ représente un groupe alkyle en $C_1$-$C_4$ ou alcoxy en $C_1$-$C_4$,
$R^9$ représente un groupe alcoxy en $C_1$-$C_4$, et
Q représente l'oxygène ou le soufre, ou bien
$R^6$ représente le groupement -$(CH_2)_n$-$R^{10}$ dans lequel
n est égal à 0, 1 ou 2, et
$R^{10}$ représente un radical hétéroxyclique éventuellement substitué par le fluor, le chlore, le brome et/ou des groupes alkyle en $C_1$-$C_4$ et choisi parmi les radicaux furyle, tétrahydrofuryle, oxotétrahydrofuryle, thiényle, tétrahydrothiényle, perhydropyrannyle, oxazolyle, thiazolyle, thiadiazolyle, dioxolannyle, perhydropyrrolyle, oxoperhydropyrrolyle, pyridinyle ou pyrimidinyle,
et leurs isomères optiques.

3. Dérivés d'acides (6-(hétéro)aryloxy-naphtalène-2-yl-oxy)-alcane-carboxyliques de formule I de la revendication 1, dans laquelle
   - $R^1$ représente un groupe cyano, le fluor ou le chlore,
   - $R^2$ représente l'hydrogène, le fluor ou le chlore,
   - $R^3$ représente le chlore ou un groupe trifluorométhyle,
   - $R^4$ représente l'hydrogène, le fluor ou le chlore,
   - X représente l'azote ou le groupement C-$R^5$ dans lequel
     $R^5$ représente l'hydrogène, le fluor ou le chlore,
   - A représente un groupe méthylène (-$CH_2$-), diméthylène (-$CH_2CH_2$), triméthylène (-$CH_2$-$)_3$ ou éthylidène

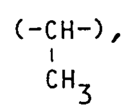

et

Z représente un groupe cyano ou le groupement -CO-Y dans lequel

Y représente le chlore, un groupe hydroxy, amino, alkylamino en $C_1$-$C_4$, phénylamino, (alcoxy en $C_1$-$C_4$)-carbonyl-alkylamino en $C_1$-$C_2$, di-(alkyle en $C_1$-$C_3$)-amino, diallylamino, alkylsulfonylamino $C_1$-$C_4$, phénylsulfonylamino, hydroxyamino, cyanamino, alcoxyamino en $C_1$-$C_4$, hydrazino, (alkyle en $C_1$-$C_4$)-sulfonylhydrazino, phénylsulfonylhydrazino, alkylthio en $C_1$-$C_4$ ou (alcoxy en $C_1$-$C_4$)-carbonyl-alkylthio en $C_1$-$C_2$, ou le groupement -O-$R^6$ dans lequel

$R^6$ représente un groupe alkyle en $C_1$-$C_4$, (alcoxy en $C_1$-$C_2$)-alkyle en $C_1$-$C_2$, (alkylthio en $C_1$-$C_2$)-alkyle en $C_1$-$C_2$, (alkyle en $C_1$-$C_2$)-sulfinylalkyle en $C_1$-$C_2$, (alkyle en $C_1$-$C_2$)-sulfonyl-alkyle en $C_1$-$C_2$, benzyloxyalkyle en $C_1$-$C_3$, benzylthio-alkyle en $C_1$-$C_3$, (alcoxy en $C_1$-$C_4$)-carbonyl-alkyle en $C_1$-$C_2$, (alkylamino en $C_1$-$C_4$)-carbonyl-alkyle en $C_1$-$C_2$, benzyle, triméthyl-silylméthyle ou un équivalent d'ammonium, d'alkylammonium en $C_1$-$C_3$, de sodium ou de potassium, ou le groupement

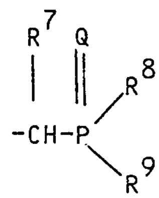

dans lequel

$R^7$ représente l'hydrogène, un groupe méthyle, phényle, furyle, thiényle ou pyridyle,

$R^8$ représente un groupe méthoxy ou éthoxy,

$R^9$ représente un groupe méthoxy ou éthoxy, et

Q représente l'oxygène ou le soufre, ou bien

$R^6$ représente le groupement $(-CH_2-)_n-R^{10}$ 10 dans lequel

n est égal à 0, 1 ou 2, et

$R^{10}$ représente un radical hétérocyclique éventuellement substitué par le chlore et/ou des groupes méthyle et choisi parmi les radicaux furyle, tétrahydrofuryle, thiényle, perhydropyrannyle, oxazolyle, thiazolyle et dioxolannyle,

**4.** Procédé de préparation des dérivés d'acides (6-(hétéro)aryloxy-naphtalène-2-yl-oxy)-alcane-carboxyliques de formule I

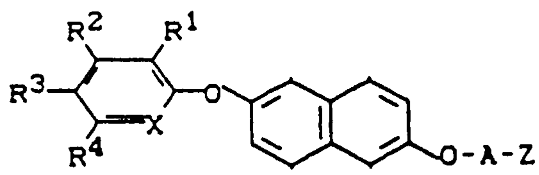

(I)

dans laquelle

$R^1$ représente l'hydrogène, un halogène, un groupe cyano ou trifluorométhyle,

$R^2$ représente l'hydrogène ou un halogène,

$R^3$ représente un halogène, un groupe trifluorométhyle, trifluorométhoxy, trifluorométhylthio ou trifluorométhylsulfonyle,

$R^4$ représente l'hydrogène ou un halogène,

X représente l'azote ou le groupe C-$R^5$ dans lequel $R^5$ représente l'hydrogène ou un halogène,

A représente un groupe alcane-diyle éventuellement ramifié, et

Z représente un groupe cyano ou le groupement -CO-Y dans lequel

Y représente un halogène, un groupe hydroxy, amino, alkylamino, alcénylamino, alcynylamino, arylamino, aralkylamino, alcoxycarbonylamino, cyanamino, dialkylamino, dialcénylamino, alkylsulfonylamino, arylsulfonylamino, hydroxyamino, alcoxyamino, hydrazino, alkylsulfonylhydrazino, arylsulfonylhydrazino,

alkylthio, arylthio, aralkylthio, alcoxycarbonylalkylthio ou le groupement -O-$R^6$ dans lequel $R^6$ représente un radical, éventuellement substitué par des halogènes, choisi parmi les radicaux alkyle, alcényle, alcynyle, alcoxyalkyle, alkythio-alkyle, alkylsulfinylalkyle, alkylsufonylalkyle, aryloxyalkyle, arylthio-alkyle, arylalcoxyalkyle, arylalkylthioalkyle, trialkylsilylalkyle, alcoxycarbonylalkyle, alkylaminocarbonylalkyle,arylaminocarbonylalkyle, N-alkyl-N-arylaminocarbonylalkyle, aralkyl, azolylalkyle, alkylidène-amino ou un équivalent d'ammonium, d'alkylammonium, de métal alcalin ou alcalino-terreux, ou bien le groupement

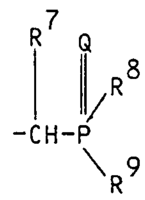

dans lequel
$R^7$ représente l'hydrogène, un groupe alkyle, aryle,furyle, thiényle ou pyridyle,
$R^8$ représente un groupe alkyle ou alcoxy,
$R^9$ représente un groupe alcoxy, et
Q représente l'oxygène ou le soufre, ou bien
$R^6$ représente le groupement $-(CH_2)_n-R^{10}$ dans lequel
$R^{10}$ représente un radical hétérocyclique éventuellement substitué par des halogènes et/ou des groupes alkyle et choisi parmi les radicaux furyle, tétrahydrofuryle, oxotétrahydrofuryle, thiényle, tétrahydrothiényle, perhydropyrannyle, oxazolyle, thiazolyle,thiadiazolyle, dioxolannyle, perhydropyrrolyle, oxoperhydropyrrolyle, pyridinyle ou pyrimidinyle, et
n est égal à 0, 1 ou 2,
caractérisé en ce que :

a) on fait réagir des 6-(hétéro)aryloxy-2-naphtols de formule générale II

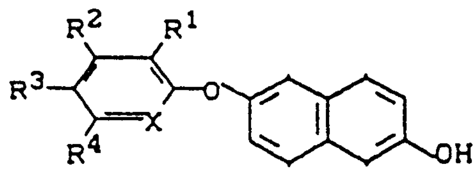

(II)

dans laquelle $R^1$, $R^2$, $R^3$, $R^4$ et X ont les significations indiquées ci-dessus,
avec des dérivés d'acides carboxyliques de formule général III

$Z^1$ - A - Z (III)

dans laquelle A et Z ont les significations indiquées ci-dessus, et
$Z^1$ représente un groupe éliminable nucléophile,
éventuellement en présence d'un accepteur d'acide et éventuellement en présence d'un diluant, ou bien

b) on fait réagir des dérivés halogéno-(hétéro)aryliques de formule générale IV

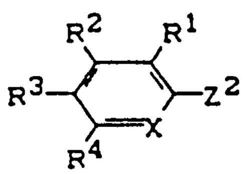

(IV)

dans laquelle $R^1$, $R^2$, $R^3$, $R^4$ et X ont les significations indiquées ci-dessus, et $Z^2$ représente un halogène,
avec des dérivés d'acides (6-hydroxynaphtalène-2-yl-oxy)-alcane-carboxyliques de formule générale V

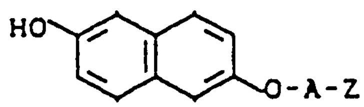

(V)

dans laquelle A et Z ont les significations indiquées ci-dessus, éventuellement en présence d'un accepteur d'acide, et éventuellement en présence d'un diluant, ou bien
c) dans les cas où, dans la formule I, Z représente le groupement -CO-Y dans lequel Y représente un groupe hydroxy, et A, $R^1$, $R^2$, $R^3$ , $R^4$ et X ont les significations indiquées ci-dessus, on fait réagir des composés de formule I dans laquelle Z représente un groupe cyano ou le groupement -CO-Y dans lequel Y représente un groupe méthoxy ou éthoxy, et A, $R^1$, $R^2$, $R^3$, $R^4$ et Y ont les significations indiquées ci-dessus, avec un hydroxyde alcalin en présence d'un solvant organique puis, éventuellement après concentrations, on acidifie par un acide minéral, ou bien
d) dans les cas où, dans la formule I, t représente le groupement -CO-Y dans lequel Y représente un halogène, et A, $R^1$, $R^2$, $R^3$, $R^4$ et X ont les significations indiquées ci-dessus, on fait réagir des composés de formule générale I dans laquelle Z représente le groupement -CO-Y dans lequel Y représente un groupe hydroxy, et A, $R^1$, $R^2$, $R^3$, $R^4$ et X ont les significations indiquées ci-dessus, avec un agent halogénant, éventuellement en présence d'un diluant et éventuellement en présence d'un catalyseur, ou bien
e) dans les cas où, dans la formule I, Z représente le groupement -CO-Y dans lequel Y a les significations indiquées ci-dessus, à l'exception d'un halogène, et A, $R^1$ , $R^2$ , $R^3$ , $R^4$ et X ont les significations indiquées ci-dessus, on fait réagir des composés de formule générale I danslaquelle Z représente le groupement -CO-Y dans lequel Y représente un halogène et A, $R^1$, $R^2$, $R^3$, $R^4$ et X ont les significations indiquées ci-dessus,
avec des composés de formule générale VI

H - Y (VI)

dans laquelle Y a les significations indiquées ci-dessus à l'exception d'un halogène, éventuellement en présence d'un accepteur d'acide et éventuellement en présence d'un diluant, ou bien
f) dans les cas où, dans la formule I, t représente le groupement -CO-Y, dans lequel Y représente le groupement -O-$R^6$, dans lequel $R^6$ a les significations indiquées ci-dessus à l'exception de l'ammonium, d'un alkylammonium, d'un métal alcalin ou alcalinoterreux, et A, $R^1$, $R^2$, $R^3$, $R^4$ et X ont tes significations indiquées ci-dessus, on fait réagir des composés de formule générale I dans laquelle Z représente le groupement -CO-Y dans lequel Y représente un groupe hydroxy et A, $R^1$, $R^2$, $R^3$, $R^4$ et Y ont les significations indiquées ci-dessus,
avec des composés de formule générale VII

$Z^3$ - $R^{6-1}$ (VII )

dans laquelle
$R^{6-1}$ a les significations indiquées ci-dessus pour $R^6$ à l'exception de l'ammonium, d'un alkylammonium, d'un métal alcalin ou alcalinoterreux, et
$Z^3$ représente un halogène,
éventuellement en présence d'un accepteur d'acide et éventuellement en présence d'un diluant, ou bien
g) dans les cas où, dans la formule I, Z représente le groupement -CO-Y, dans lequel Y représente le groupement -O-$R^6$, dans lequel $R^6$ a les significations indiquées ci-dessus à l'exception de l'ammonium, d'un alkylammonium, d'un métal alcalin ou alcalinoterreux, et A, $R^1$ $R^2$ , $R^3$ $R^4$ et X ont les significations indiquées ci-dessus, on fait réagir des composés de formule I dans laquelle Z représente un groupe cyano et A, $R^1$, $R^2$, $R^3$, $R^4$ ont les significations indiquées ci-dessus,
avec des dérivés hydroxylés de formule générale VIII

HO-$R^{6-2}$ (VIII)

dans laquelle
$R^{6-2}$ représente un groupe alkyle en $C_1$-$C_6$, alcényle en $C_3$-$C_4$,alcynyle en $C_3$-$C_4$, (alcoxy en $C_1$-$C_4$)-alkyle en $C_1$-$C_4$, (alkylthio en $C_1$-$C_4$)-alkyle en $C_1$-$C_4$, phénoxy-alkyle en $C_1$-$C_3$, phénylthio-alkyle en $C_1$-$C_3$, benzyloxy-alkyle en $C_1$-$C_3$, benzylthio-alkyle en $C_1$-$C_3$, et (alcoxy en $C_1$-$C_4$)-carbonyl-alkyle en $C_1$-$C_2$,
en présence d'un acide minéral et le cas échéant en présence d'un diluant.

**5.** Produits herbicides, caractérisés en ce qu'ils contiennent au moins un dérivé d'acide (6-(hétéro)aryloxy-naphtalène-2-yl-oxy)-alcane-carboxylique de formule I selon revendications 1 à 4.

**6.** Procédé pour combattre les végétaux adventices, caractérisé en ce que l'on fait agir sur ces végétaux et/ou leur habitat des dérivés d'acides (6-(hétéro)aryloxy-naphtalène-2-yl-oxy)-alcane-carboxyliques de formule I selon revendications 1 à 4.

**7.** Utilisation des dérivés d'acides (6-(hétéro)aryloxynaphtalène-2-yl-oxy)-alcane-carboxyliques de formule I des revendications 1 à 4, pout la lutte contre les végétaux adventices.

**8.** Procédé de préparation de produits herbicides, caractérisé en ce que l'on mélange des dérivés d'acides (6-(hétéro)aryloxy-naphtalène-2-yl-oxy)-alcane-carboxyliques de formule I des revendications 1 à 4 avec des diluants et/ou des agents tensioactifs.

**9.** 6-(hétéro)aryloxy-2-naphtols de formule II

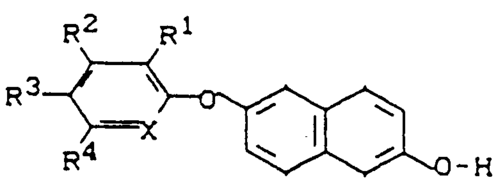

(II)

dans laquelle
$R^1$ représente l'hydrogène, un halogène, un groupe cyano ou trifluorométhyle,
$R^2$ représente l'hydrogène ou un halogène,
$R^3$ représente un halogène, un groupe trifluorométhyle, trifluorométhoxy, trifluorométhylthio ou trifluorométhylsulfonyle,
$R^4$ représente l'hydrogène ou un halogène, et
X représente l'azote ou le groupement C-$R^5$ dans lequel
$R^5$ représente l'hydrogène ou un halogène.

**10.** procédé de préparation des 6-(hétéro)aryloxy-2-naphtols de formule II

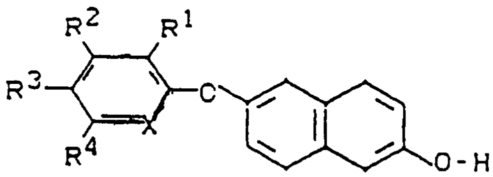

(II)

dans laquelle
$R^1$ représente l'hydrogène, un halogène, un groupe cyano ou tri fluorométhyle,
$R^2$ représente l'hydrogène ou un halogène,

$R^3$ représente un halogène, un groupe trifluorométhyle, trifluorométhoxy, trifluorométhylthio ou trifluorométhylsulfonyle,
$R^4$ représente l'hydrogène ou un halogène, et
X représente l'azote ou le groupement C-$R^5$ dans lequel
$R^5$ représente l'hydrogène ou un halogène.
caractérisé en ce que l'on fait réagir des dérivés halogéno-(hétéro)aryliques de formule IV

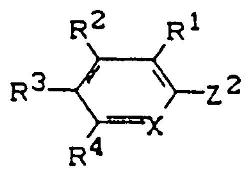
(IV)

dans laquelle $R^1$, $R^2$, $R^3$, $R^4$ et X ont les significations ci-dessus, et $Z^2$ représente un halogène, avec le 2,6-dihydroxynaphtalène en présence d'un accepteur d'acide et en présence d'un diluant.